# EUROPÄISCHE PATENTSCHRIFT

(11) **EP 2 069 288 B1**
(45) Veröffentlichungstag und Bekanntmachung des Hinweises auf die Patenterteilung: **26.03.2014**
(21) Anmeldenummer: 07802030.2
(22) Anmeldetag: 31.08.2007
(51) Int. Cl.: C07C 235/16, C07C 235/24, C07C 257/18, C07D 217/22, C07D 235/30, A61K 31/165, A61K 31/4184, A61P 7/02

(54) **TARTRATDERIVATE ALS INHIBITOREN DES KOAGULATIONSFAKTORS IXa**
TARTRATE DERIVATIVES FOR USE AS COAGULATION FACTOR IXa INHIBITORS
DÉRIVÉS DE TARTRATE UTILISÉS COMME INHIBITEURS DU FACTEUR DE COAGULATION IXa

(30) Priorität: 13.09.2006 DE 102006042927
(43) Veröffentlichungstag der Anmeldung: 17.06.2009
(73) Patentinhaber: SANOFI, 75008 Paris (FR)
(72) Erfinder: STEINHAGEN, Henning, 65926 Frankfurt am Main (DE); FOLLMANN, Markus, 42489 Wülfrath (DE); GOERLITZER, Jochen, 65926 Frankfurt am Main (DE); SCHREUDER, Herman, 65926 Frankfurt am Main (DE)
(74) Vertreter: Then, Johann
(86) Internationale Anmeldenummer: PCT/EP2007/007612
(87) Internationale Veröffentlichungsnummer: WO 2008/031508

(56) Entgegenhaltungen:
- EP-A- 0 308 364
- WO-A-94/22885
- DE-A1- 10 104 598

## Beschreibung

Die Erfindung betrifft neue Verbindungen der formel I mit antithrombotischer Aktivität, die insbesondere den Blutgerinnungsfaktor IXa inhibieren, Verfahren zu ihrer Herstellung und Verwendung derselben als Arzneimittel.

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der Vasokonstriktion oder Gefäßkontraktion: Hierdurch wird der Blutverlust in das geschädigte Areal vermindert.
2. Die nächste Phase ist die der Plättchenaktivierung durch Thrombin. Die Plättchen heften sich an der Stelle des Gefäßwandschadens an und bilden ein Plättchenaggregat. Das Protein Fibrinogen ist hierbei für die Quervernetzung der Plättchen über entsprechende Oberflächenrezeptoren verantwortlich. Plättchen binden auch an freigelegtes Kollagen der extrazellulären Matrix der geschädigten Gefäßwand und werden hierdurch aktiviert. Nach Aktivierung der Plättchen wird eine Reihe von Botenstoffen ausgeschüttet, die die Aktivierung von weiteren Plättchen induzieren. Gleichzeit wird ein Membranlipid, Phosphatidylserin, von der Membraninnenseite der Plättchen auf die Außenseite transportiert, an das sich Komplexe aus Gerinnungsfaktoren anlagern können. Die Plättchen beschleunigen über diesen Mechanismus die Blutgerinnung.
3. Die Bildung dieser Gerinnungskomplexe führt zur massiven Bildung von Thrombin welches das lösliche Fibrinogen durch Abspaltung zweier kleiner Peptide in Fibrin überführt. Fibrinmonomere bilden spontan fadenförmig Stränge aus denen nach Quervernetzung durch den Gerinnungsfaktor XIII ein stabiles Proteinnetz bildet. Das anfänglich noch lockere Plättchenaggregat wird durch dieses Fibrinnetz stabilisiert, Plättchenaggregate und Fibrinnetz sind die beiden essentiellen Bestandteile eines Thrombus.
4. Nach Wundheilung wird der Thrombus durch die Einwirkung des Schlüsselenzyms des körpereigenen Fibrinolysesystems Plasmin aufgelöst.

Zwei alternative Wege können zur Bildung eines Fibringerinnsels führen, der intrinsische und der extrinsische Weg. Diese Wege werden durch unterschiedliche Mechanismen eingeleitet, in späterer Phase konvergieren sie jedoch zu einer gemeinsamen Endstrecke der Gerinnungskaskade. In dieser Endstrecke der Gerinnung wird der Gerinnungsfaktor X aktiviert. Der-aktivierte Faktor X ist für die Bildung von Thrombin aus der im Blut zirkulierenden inaktiven Vorstufe Prothrombin verantwortlich: Die Bildung eines Thrombus auf dem Boden einer Gefäßwandabnormität ohne Wunde istdas Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren V, VIII, IX, X, XI und XII sowie Präkallikrein, hochmolekulares Kininogen, Calciumionen und Phospholipide aus Plättchen.

Der intrinsische Weg wird eingeleitet, wenn Präkallikrein, hochmolekulares Kininogen Faktor XI und XII an eine negativ geladene Oberfläche binden. Dieser Zeitpunkt wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallikrein in Kallikrein, das wiederum den Faktor XII aktiviert. Faktor XIIa hydrolisiert weiteres Präkallikrein zu Kallikrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwsenheit von Ca²⁺-Ionen aktiviert der Faktor XIa den Faktor IX. Faktor IX ist ein Proenzym, das Vitamin-K abhängige, γ-Carboxyglutaminsäure (GLA)-Reste enthält. Die Serinproteaseaktivität kommt nach Bindung von Ca²⁺ an diese GLA-Reste zum Tragen. Mehrere der Serinproteasen der Blutgerinnungs-kaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Tenasekomplexes aus Ca²⁺ und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Tenasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Tenasekomplexbildung und damit zu einer Eingrenzung der Blutgerinnung.

Der extrinsische Weg erfordert einen Gewebefaktor TF (Tissue Faktor) und die Gerinnungsfaktoren V, VII, VIII, IX und X. Bei einer Gefäßverletzung kommt der Gewebefaktor TF (Tissue Faktor) mit dem Gerinnungsfaktor VII zusammen und dieser wird aktiviert. Der Komplex aus TF und dem Gerinnungsfaktor VII hat zwei Substrate, die Gerinnungsfaktoren X und IX.

Der Gerinnungsfaktor IX kann über den intrisischen Weg und den extrinsischen Weg aktiviert werden. Die Aktivierung von Faktor IXa stellt somit eine zentrale Schnittstelle zwischen beiden Wegen der Gerinnungsaktivierung dar.

Der Faktor IXa hat eine wichtige Rolle in der Blutgerinnung. Defekte am Faktor IXa führen zu Hämophilie B, während erhöhte Konzentrationen von Faktor IXa im Blut zu einem signifikant erhöhten Risiko von Thrombosebildung führen (Weltermann A, et al., J Thromb Haemost. 2003; 1: 28-32). Die Regulation der Faktor IXa Aktivität kann die Thrombusbildung in Tiermodellen reduzieren (Feuerstein GZ, et al., Thromb Haemost. 1999; 82: 1443-1445).

Bestimmte Carbonsäureamide, deren Herstellung und deren Verwendung als Arzneimittel werden in der Offenlegungsschrift DE 10104598 A1 und bestimmte Faktor IXa Inhibitoren werden in der Internationalen Anmeldung WO 94/22885 beschrieben.

Die erfindungsgemäßen Verbindungen der Formel I eignen sich für die prophylaktische als auch für die therapeutische Anwendung am Menschen, die an Erkrankungen leiden, die mit Thrombosen, Embolien, Hyperkoagulabilität oder fibrotischen Veränderungen einhergehen. Sie können zur Sekundär-Prävention eingesetzt werden und eignen sich sowohl für eine akute als auch für eine Langzeittherapie.

Die Erfindung betrifft daher eine Verbindung der Formel I 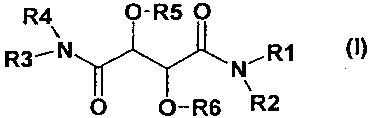 und/oder alle stereoisomeren Formen der Verbindung der Formel I und/oder Gemische dieser Formen in jedem Verhältnis, und/oder ein physiologisch verträgliches Salz der Verbindung der Formel I, wobei
- R1 für: 1) -(C₆-C₁₄)-Aryl-Z, worin Z eine basische stickstoffhaltige Gruppe bedeutet, wobei die konjugierte Säure dieser Gruppe einen pKa von 5 bis 15 hat und worin Aryl unsubstituiert oder ein-, zwei- oder dreifach durch T substituiert ist,
2) ein vier- bis fünfzehngliedrigen Het-Z, worin Z eine basische stickstoffhaltige Gruppe bedeutet, wobei die konjugierte Säure dieser Gruppe einen pKa von 5 bis 15 hat und worin Het unsubstituiert oder zusätzlich ein-, zwei- oder dreifach durch T substituiert ist, steht,
- R2 und R4: gleich sind und jeweils Wasserstoffatom bedeuten,
- R3 für: 1) -(C₀-C₄)-Alkylen-(C₆-C₁₄)-Aryl, worin Aryl unsubstituiert oder ein-, zwei- oder dreifach durch T substituiert ist,
2) -(C₀-C₄)-Alkylen-(C₆-C₁₄)-Aryl-Q-(C₆-C₁₄)-Aryl, worin die beiden Aryle jeweils unabhängig voneinander unsubstituiert oder ein-, zwei- oder dreifach durch T substituiert sind, oder
3) -(C₀-C₄)-Alkylen-(C₆-C₁₄)-Aryl-Q-Het, worin Aryl und Het jeweils unabhängig voneinander unsubstituiert oder ein-, zwei- oder dreifach durch T substituiert sind, steht,
- Q für: kovalente Bindung, -(C₁-C₄)-Alkylen, -NH-, -N((C₁-C₄)-Alkyl)-, -O-, -SO₂- oder -S- steht,
- T für: 1) Halogen,
2) -(C₁-C₆)-Alkyl, worin Alkyl unsubstituiert oder unabhängig voneinander ein, zwei- oder dreifach durch -(C₁-C₃)-Fluoralkyl, -N-C(O)-OH oder -N-C(O)-(C₁-C₄)-Alkyl substituiert sind,
3) -(C₃-C₈)-Cycloalkyl oder
4) -NO₂, steht,
- R5 und R6: gleich sind und jeweils für Wasserstoffatom stehen.

Die Erfindung betrifft ferner eine Verbindung der Formel I
und/oder alle stereoisomeren Formen der Verbindung der Formel I und/oder Gemische dieser Formen in jedem Verhältnis, und/oder ein physiologisch verträgliches Salz der Verbindung der Formel I, wobei
- R1 für: 1) -(C₆-C₁₄)-Aryl-Z, wobei Aryl ausgewählt ist aus der Gruppe Phenyl und Naphthyl, und worin Aryl unsubstituiert oder ein-, zwei- oder dreifach durch T substituiert ist und Z Amino, Aminomethylen, Amidino, Guanidino, Azetidinyl, Pyrrolidinyl, Piperidinyl, Pyridinyl oder Aminopyridinyl bedeutet, oder
2) ein vier- bis fünfzehngliedrigen Het-Z, wobei Het ausgewählt ist aus der Gruppe Acridinyl, Azepinyl, Azetidinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, beta-Carbolinyl, Chinazolinyl, Chinolinyl, Chinolizinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, Dioxolenyl, 2H, 6H-1,5,2-Dithiazinyl, Furanyl, Furazanyl, lmidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Phenanthridinyl, Phenanthrenyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazinyl, Thiazolyl, Thienyl, Thienoimidazolyl, Thienooxazolyl, Thienopyridinyl, Thienopyrrolyl, Thienothiazolyl, Thienothiophenyl, Thiomorpholinyl, Thiopyranyl, Triazinyl, 1,2,3-Triazolyl, 1,2,4-Triazolyl, 1,2,5-Triazolyl, 1,3,4-Triazolyl oder Xanthenyl und worin Het unsubstituiert oder ein-, zwei- oder dreifach durch T substituiert ist und worin Z wie oben definiert ist, steht,
- R2 und R4: gleich sind und jeweils Wasserstoffatom bedeuten,
- R3 für: 1) -(C₀-C₄)-Alkylen-(C₆-C₁₄)-Aryl, worin Aryl wie oben definiert ist und unsubstituiert oder ein-, zwei- oder dreifach durch T substituiert ist,
2) -(C₀-C₄)-Alkylen-(C₆-C₁₄)-Aryl-Q-(C₆-C₁₄)-Aryl, worin die beiden Aryle jeweils unabhängig voneinander wie oben definiert sind und jeweils unabhängig voneinander unsubstituiert oder ein-, zwei- oder dreifach durch T substituiert sind, oder
3) -(C₀-C₄)-Alkylen-(C₆-C₁₄)-Aryl-Q-Het, worin Aryl und Het wie oben definiert sind und jeweils unabhängig voneinander unsubstituiert oder ein-, zwei- oder dreifach durch T substituiert sind, steht
- Q für: kovalente Bindung, -(C₁-C₄)-Alkylen, -NH-, -N((C₁-C₄)-Alkyl)- oder -O- steht,
- T für: 1) Halogen,
2) -(C₁-C₆)-Alkyl, worin Alkyl unsubstituiert oder unabhängig voneinander ein, zwei- oder dreifach durch -(C₁-C₃)-Fluoralkyl, -N-C(O)-OH oder -N-C(O)-(C₁-C₄)-Alkyl substituiert ist,
3) -(C₃-C₆)-Cycloalkyl oder
4) -NO₂, steht,
- R5 und R6: gleich sind und jeweils für Wasserstoffatom stehen.

Die Erfindung betrifft ferner eine Verbindung der Formel I und/oder alle stereoisomeren Formen der Verbindung der Formel I und/oder Gemische dieser Formen in jedem Verhältnis, und/oder ein physiologisch verträgliches Salz der Verbindung der Formel I, wobei
- R1 für: Carbamimidoyl-phenyl, Aminomethylphenyl oder Het-Z steht, wobei Het ausgewählt ist aus der Gruppe Benzimidazolyl und Isochinolinyl, und worin Z für Amino oder Amidino steht,
- R2 und R4: jeweils für Wasserstoffatom stehen,
- R3 für: 1) Phenyl, worin Phenyl unsubstituiert oder ein- oder zweifach durch T substituiert ist,
2) -Phenyl-Q-Phenyl, worin die beiden Phenylreste jeweils unabhängig voneinander unsubstituiert oder ein- oder zweifach durch T substituiert sind, oder
3) Phenyl-Q-Het-2, worin Het-2 ausgewählt ist aus der Gruppe Chinolinyl, Chinoxalinyl, Furanyl, Indolyl, Isochinolinyl, Isothiazolyl, Isoxazolyl, Oxadiazolyl, Piperidinyl, Pyrazolyl, Pyridazinyl, Pyrimidinyl, Pyridyl, Pyrimidinyl, Pyrrolyl, Thiadiazolyl, Thiazolyl, Thienyl, Thienopyrrol oder Thienothiophenyl, und worin Phenyl und Het-2 jeweils unabhängig voneinander unsubstituiert oder ein- oder zweifach durch T substituiert sind, steht,
- Q für: kovalente Bindung, -CH₂-, -N(CH₃)- oder -O- steht,
- T für: 1) F, Cl oder Br,
2) -(C₁-C₄)-Alkyl, worin Alkyl unsubstituiert oder unabhängig voneinander ein- oder zweifach durch -CF₃ oder -N-C(O)-CH₃ substituiert sind, oder
3) -NO₂, steht, und
- R5 und R6: jeweils Wasserstoffatom bedeuten.

Ein weiterer Gegenstand der Erfindung sind Verbindungen der Formel I aus der Reihe (2R, 3R)-N-(1-Amino-isochinolin-6-yl)-2,3-dihydroxy-N'-p-tolyl-bernsteinsäure-amid,
(2R, 3R)-N-(2-Amino-3H-benzimidazol-5-yl)-2,3-dihydroxy-N'-p-tolyl-bernsteinsäure-amid,
(2R, 3R)-N-(2-Amino-3H-benzimidazol-5-yl)-N'-(4-cyclohexyl-phenyl)-2,3-dihydroxybernsteinsäure-amid,
(2R,3R)-N-(4-Aminomethyl-phenyl)-2,3-dihydroxy-N'-p-tolyl-bersteinsäure-amid,
(2R,3R)-N-(4-Carbamimidoyl-phenyl)-2,3-dihydroxy-N'-p-tolyl-bernsteinsäure-amid,
(2R, 3R)-N-(2-Amino-3H-benzimidazol-5-yl)-N'-(4-fluorphenyl)-2,3-dihydroxybernsteinsäure-amid,
(2R, 3R)-N-(2-Amino-3H-benzimidazol-5-yl)-N'-(4-chlorphenyl)-2,3-dihydroxybernsteinsäure-amid,
(2R, 3R)-N-(2-Amino-3H-benzimidazol-5-yl)-2,3-dihydroxy-N'-phenyl-bernsteinsäure-amid,
(2R, 3R)-N-(2-Amino-3H-benzimidazol-5-yl)-2,3-dihydroxy-N'-(4-nitro-phenylbernsteinsäure-amid,
(2R, 3R)-N-(2-Amino-3H-benzimidazol-5-yl)-2,3-dihydroxy-N'-(4-isopropyl-phenylbernsteinsäure-amid,
(2R, 3R)-N-(2-Amino-3H-benzimidazol-5-yl)-2,3-dihydroxy-N'-(4-piperidin-1-yl-phenyl)-bernsteinsäure-amid oder
(2R, 3R)-N-(2-Amino-3H-benzimidazol-5-yl)-2,3-dihydroxy-N'-(4-phenoxy-phenyl)-bernsteinsäure-amid ist.

Unter dem Begriff "(C₁-C₄)-Alkyl" oder "(C₁-C₆)-Alkyl" werden Kohlenwasserstoffreste verstanden, deren Kohlenstoffkette geradkettig oder verzweigt ist und 1 bis 4
- R12: -(C₁-C₆)-Alkyl, -(C₃-C₈)-Cycloalkyl, -(C₆-C₁₄)-Aryl oder Het bedeutet; und worin Aryl und Het wie oben definiert sind.

Die Erfindung betrifft ferner eine Verbindung der Formel und/oder alle stereoisomeren Formen der Verbindung der Formel I und/oder Gemische dieser Formen in jedem Verhältnis, und/oder ein physiologisch verträgliches Salz der Verbindung der Formel I, wobei
- R1 für: Carbamimidoyl-phenyl (Benzamidino), Aminomethylphenyl oder Het-Z steht, wobei Het ausgewählt ist aus der Gruppe Benzimidazolyl und Isochinolinyl, und worin Z für Amino oder Amidino steht,
- R2 und R4: jeweils für Wasserstoffatom stehen,
- R3 für: 1) Phenyl, worin Phenyl unsubstituiert oder ein- oder zweifach durch T substituiert ist,
2) -Phenyl-Q-Phenyl, worin die beiden Phenylreste jeweils unabhängig voneinander unsubstituiert oder ein- oder zweifach durch T substituiert sind,
3) Phenyl-Q-(C₃-C₆)-Cycloalkyl, worin Phenyl und Cycloalkyl jeweils unabhängig voneinander unsubstituiert oder ein- oder zweifach durch T substituiert sind, oder
4) Phenyl-Q-Het-2, worin Het-2 ausgewählt ist aus der Gruppe Chinolinyl, Chinoxalinyl, Furanyl, Indolyl, Isochinolinyl, Isothiazolyl, Isoxazolyl, Oxadiazolyl, Piperidinyl, Pyrazolyl, Pyridazinyl, Pyrimidinyl, Pyridyl, Pyrimidinyl, Pyrrolyl, Thiadiazolyl, Thiazolyl, Thienyl, Thienopyrrol oder Thienothiophenyl und worin Phenyl und Het-2 jeweils unabhängig voneinander unsubstituiert oder ein- oder zweifach durch T substituiert sind, steht,
- Q für: kovalente Bindung, -CH₂-, -N(CH₃)- oder -O- steht,
- T für: 1) F, Cl oder Br,
2) -(C₁-C₄)-Alkyl, worin Alkyl unsubstituiert oder unabhängig voneinander ein- oder zweifach durch -CF₃ oder -N-C(O)-CH₃ substituiert sind,
3) -CF₃,
4) -O-(C₁-C₄)-Alkyl,
5) -O-CF₃,
6) -NO₂,
7) -N(R10)(R11), worin R10 und R11 unabhängig voneinander Wasserstoffatom oder -(C₁-C₄)-Alkyl bedeuten, oder
8) -SO₂-CH₃, steht,
- R5 und R6: jeweils Wasserstoffatom bedeuten.

Ein weiterer Gegenstand der Erfindung sind Verbindungen der Formel I aus der Reihe
(2R, 3R)-N-(-1-Amino-isochinolin-6-yl)-2,3-dihydroxy-N'-p-tolyl-bernsteinsäureamid
(2R, 3R)-N-(2-Amino-3H-benzimidazol-5-yl)-2,3-dihydroxy-N'-p-tolyl-bernsteinsäureamid,
(2R, 3R)-N-(2-Amino-3H-benzimidazol-5-yl)-N'-(4-cyclohexyl-phenyl)-2,3-dihydroxybernsteinsäureamid,
(2R,3R)-N-(4-Aminomethyl-phenyl)-2,3-dihydroxy-N'-p-tolyl-bersteinsäureamid;
(2R,3R)-N-(4-Carbamimidoyl-phenyl)-2,3-dihydroxy-N'-p-tolyl-bernsteinsäureamid,
(2R, 3R)-N-(2-Amino-3H-benzimidazol-5-yl)-N'-(4-fluorphenyl)-2,3-dihydroxybernsteinsäureamid,
(2R, 3R)-N-(2-Amino-3H-benzimidazol-5-yl)-N'-(4-chlorphenyl)-2,3-dihydroxybernsteinsäureamid,
(2R, 3R)-N-(2-Amino-3H-benzimidazol-5-yl)-2,3-dihydroxy-N'-phenyl-bernsteinsäureamid,
(2R, 3R)-N-(2-Amino-3H-benzimidazol-5-yl)-2,3-dihydroxy-N'-(4-nitro-phenylbernsteinsäureamid,
(2R, 3R)-N-(2-Amino-3H-benzimidazol-5-yl)-2,3-dihydroxy-N'-(4-isopropyl-phenylbernsteinsäureamid,
(2R, 3R)-N-(2-Amino-3H-benzimidazol-5-yl)-2,3-dihydroxy-N'-(4-piperidin-1-yl-phenyl)bernsteinsäureamid oder
(2R, 3R)-N-(2-Amino-3H-benzimidazol-5-yl)-2,3-dihydroxy-N'-(4-phenoxy-phenyl)bernsteinsäureamid.

Unter dem Begriff "(C₁-C₄)-Alkyl" oder "(C₁-C₆)-Alkyl" werden Kohlenwasserstoffreste verstanden, deren Kohlenstoffkette geradkettig oder verzweigt ist und 1 bis 4 oder 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl. Unter dem Begriff "-(C₀-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-C₀-Alkylen" ist eine kovalente Bindung. Unter dem Begriff "-(C₁-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen (-CH₂-), Ethylen (-CH₂-CH₂-), Propylen (-CH₂-CH₂-CH₂-), Iso-Propylen, Iso-Butylen, Butylen oder tertiär-Butylen.

Unter dem Begriff "-(C₃-C₁₂)-Cycloalkyl" werden Ringe aus 3 bis 12 Kohlenstoffatomen verstanden wie Verbindungen, die sich von Monocyclen mit 3 bis 8 Kohlenstoffatomen im Ring wie Cyclopropan, Cyclobutan, Cyclopentan, Cyclohexan, Cycloheptan oder Cyclooctan herleiten, die sich von den Bicyclen Bicyclo[4.2.0]octan, Octahydro-inden, Decahydro-naphthalen, Decahydro-azulen, Decahydrobenzocyclohepten oder Dodecahydro-heptalen herleiten oder von den überbrücken Cyclen wie Spiro[2.5]octan, Spiro[3.4]octan, Spiro[3.5]nonan, Bicyclo[3.1.1]heptan, Bicyclo[2.2.1]heptan oder Bicyclo[2.2.2]octan herleiten.

Unter dem Begriff "-(C₃-C₆)-Cycloalkyl" oder "-(C₃-C₈)-Cycloalkyl" werden Reste verstanden, die sich von Monocyclen mit 3 bis 6 oder 3 bis 8 Kohlenstoffatomen im Ring ableiten wie Cyclopropan, Cyclobutan, Cyclopentan, Cyclohexan, Cycloheptan oder Cyclooctan.

Unter dem Begriff "-(C₆-C₁₄)-Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.

Unter dem Begriff "vier- bis fünfzehngliedrigen Het" oder "Het" werden Ringsysteme verstanden mit 4 bis 15 Kohlenstoffatomen, die in ein, zwei oder drei miteinander verbundenen Ringsystemen vorliegen und in denen ein, zwei, drei oder vier gleiche oder verschiedene Heteroatome aus der Reihe Sauerstoff, Stickstoff oder Schwefel die jeweiligen Kohlenstoffatome ersetzen können. Beispiele für diese Ringsysteme sind die Reste Acridinyl, Azepinyl, Azetidinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, beta-Carbolinyl, Chinazolinyl, Chinolinyl, Chinolizinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, Dioxolenyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Phenanthridinyl, Phenanthrenyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pyridooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazinyl, Thiazolyl, Thienyl, Thienoimidazolyl, Thienooxazolyl, Thienopyridinyl, Thienopyrrolyl, Thienothiazolyl, Thienothiophenyl, Thiomorpholinyl, Thiopyranyl, Triazinyl, 1,2,3-Triazolyl, 1,2,4-Triazolyl, 1,2,5-Triazolyl, 1,3,4-Triazolyl oder Xanthenyl.

Unter dem Begriff "-(C₁-C₃)-Fluoralkyl" wird ein partiell oder vollständig fluorierter Alkylrest verstanden, der sich beispielsweise von folgenden Resten ableitet -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF2, -CH₂-CF₂-CH₂F, -CHF-CHF-CF₃, -CHF-CHF-CHF₂, -CHF-CHF-CH₂F, -CHF-CH₂-CF₃, ₋CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F -CF₂-CH₂-CF₃, -CF₂-CH₂-CHF₂, -CF₂-CH₂-CH₂F, -CF₂-CF₂-CF₃, -CF₂-CF₂-CHF₂ oder -CF₂-CF₂-CH₂F.

Unter dem Begriff "Halogen" wird Fluor, Chlor, Brom oder Jod verstanden, bevorzugt sind Fluor, Chlor oder. Brom, insbesondere Chlor oder Brom.

Unter dem Begriff "eine basische stickstoffhaltige Gruppe" werden Reste verstanden, wobei die konjungierte Säure dieser Gruppe einen pKa von etwa 5 bis 15 hat. Beispiele für diese basische stickstoffhaltige Gruppe sind Amino, Aminomethylen, Amidino (Carbamimidoyl), Guanidino, Azetidinyl, Pyrrolidinyl, Piperidinyl, Pyridinyl oder Aminopyridinyl.

Funktionelle Gruppen der verwendeten Intermediate, beispielsweise Amino- oder Carboxylgruppen, können dabei durch geeignete Schutzgruppen maskiert werden. Geeignete Schutzgruppen für Aminofunktionen sind beispielweise die t-Butoxy-carbonyl-, die Benzyloxycarbonyl-, Phtalolyl-, die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Green, T.W., Wutz, P.G.M., Protective Groups in Organic Synthesis (1991), 2nd Ed., Wiley-Interscience, oder Kocienski, P., Protecting Groups (1994), Thieme). Der Begriff Schutzgruppe kann auch entsprechende polymergebundene Schutzgruppen umfassen. Solcherart maskierte Verbindungen gemäß Formel (I), in der beispielsweise die funktionellen Gruppen der Reste U, V, X oder W gegebenenfalls ebenfalls maskiert sein können, können, obwohl gegebenenfalls selber pharmakologisch nicht aktiv, gegebenenfalls nach Administration in Säugern durch Metabolisierung zu den erfindungsgemäßen, pharmakologisch aktiven Verbindungen umgewandelt werden.

Die erfindungsgemäßen Verbindungen können nach wohlbekannten Verfahren oder nach hier beschriebenen Verfahren hergestellt werden.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man die Verbindung der Formel I nach Schema 1 herstellt. Schema 1:

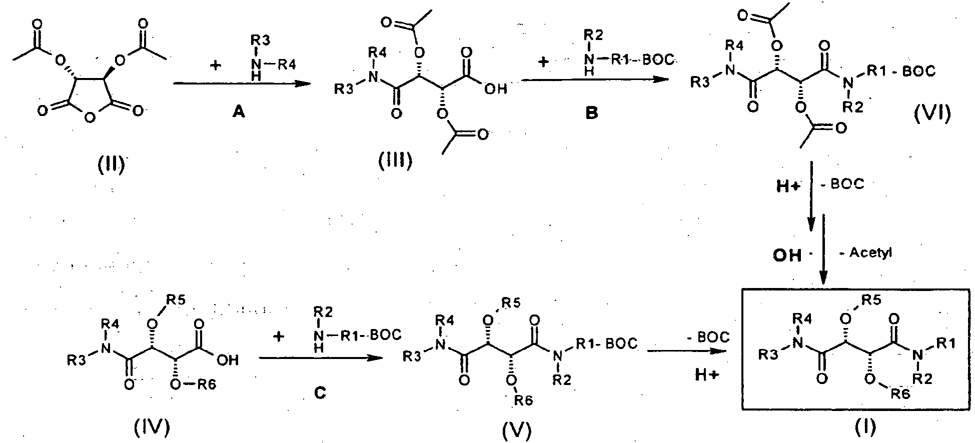

Die in Schema 1 verwendeten Reste R1, R2, R3 und R4 haben dieselbe Bedeutung wie die in der Verbindung der Formel I, BOC steht für die Schutzgruppe Butoxycarbonyl.

In einem Verfahrensschritt A wird Diacetyl-L-weinsäureanhydrid (Verbindung der Formel II) in einem Lösungsmittel wie Dimethylformamid (DMF), Tetrahydrofuran (THF), N-Methylpyrrolidinon (NMP), Dioxan oder Dichlormethan gelöst und mit einem geeigneten Amin der Formel NH(R3)-R4 zum entsprechenden Amid (III) umgesetzt. Hierzu wird eine geeignete Base wie N-Methylmorpholin oder auch eine andere Aminbase wie Hünigs Base, Triethylamin (NEt₃) oder 4-Dimethylamino-pyridin (4-DMAP) zugesetzt. Im nächsten Schritt B wird das Monoamid III in einem Lösungsmittel wie Dimethylformamid (DMF), Tetrahydrofuran (THF), NMP, Dioxan oder Dichlormethan gelöst und mit einem geeigneten Amin der Formel NH(R1-BOC)-R2 zum entsprechenden Diamid (VI) gekuppelt. Hierzu werden wie oben beschrieben ein übliches Kupplungsreagenz wie TOTU, PyBrop, PyBop, HATU oder EDC und eine geeignete Base wie Aminbasen wie Hünigs Base, NEt₃ oder DMAP verwendet.

Nach Abspaltung von Schutzgruppen wie der Boc Schutzgruppe an N(R1)-R2 mit Standard-Methoden wie mit TFA-CH₂Cl₂ und Abspaltung der Acetylgruppen durch basische Hydrolyse beispielsweise mit NaOH bei Raumtemperatur (RT) erhält man die Zielverbindungen (I). (Alternative Methoden zur Abspaltung von Schutzgruppen siehe z.B. Kocienski, P.J., Protecting groups, Thieme Verlag 1994, S. 1-16). Diese Route ergibt Verbindungen des Typs I mit R6=H. Verbindungen mit R6 ungleich H können prinzipiell aus diesen nach bekannten Standardverfahren hergestellt werden (beispielsweise Esterbildung oder Carbamoylierungen)

In einem alternativen Verfahren (C) wird direkt ein Weinsäuremonoamid (IV) in einem Lösungsmittel wie Dimethylformamid (DMF), Tetrahydrofuran (THF), NMP, Dioxan oder CH₂Cl₂ gelöst und mit einem geeigneten Amin der Formel NH(R1-B-OC)-R2 zum entsprechenden Diamid (V) gekuppelt. Hierzu werden ein übliches Kupplungsreagenz wie TOTU, PyBrop, PyBop, Hatu oder EDC und eine geeignete Base wie Aminbasen wie Hünigs Base, NEt₃ oder DMAP verwender. Nach der Abspaltung von Schutzgruppen die der BOC-Schutzgruppen an N(R1)-R2 mit Standard-Methoden, beispielsweise TFA-CH₂Cl₂ bei RT erhält man die Zielverbindungen (I). Verbindungen mit R6 ungleich H können prinzipiell auch nach diesen Verfahren hergestellt werden.

Die Erfindung betrifft ferner ein Verfahren zur Herstellung der Verbindung der Formel I und/oder einer stereoisomeren Form der Verbindung der Formel und/oder eines physiologisch verträglichen Salzes der Verbindung der Formel I, das dadurch gekennzeichnet ist, dass man
a) eine Verbindung der Formel II 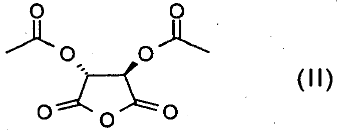 mit einer Verbindung NH(R3)(R4) zu einer Verbindung der Formel III umsetzt, 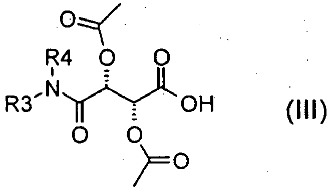 wobei die Reste R3 und R4 wie in Formel I definiert sind und die Verbindung der Formel III mit einer Verbindung NH(R2)(R1)-Boc zu einer Verbindung der Formel VI umsetzt, 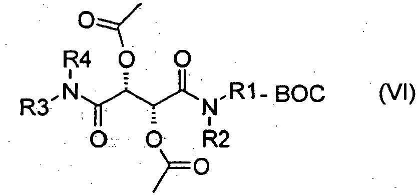 wobei die Reste R1, R2, R3 und R4 wie in Formel I definiert sind und Boc für die Schutzgruppe Butoxycarbonyl steht, und anschließend zu einer Verbindung der Formel I umsetzt, oder
b) eine Verbindung der Formel IV 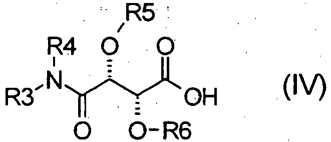 wobei die Reste R3, R4, R5 und R6 wie in Formel I definiert sind, mit einer Verbindung NH(R2)(R1)-Boc zu einer Verbindung der Formel V umsetzt, 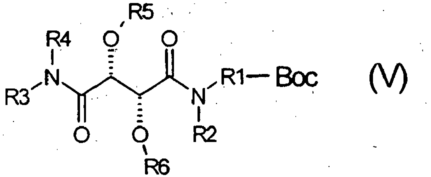 wobei die Reste R1, R2, R3, R4, R5 und R6 wie in Formel definiert sind und Boc für die Schutzgruppe Butoxycarbonyl steht, und anschließend zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

Eine nach Schema 1 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren aufgetrennt werden (Verfahren d), oder die nach Schema 1 hergestellte Verbindung der Formel I kann entweder in freier Form isoliert oder im Falle des Vorliegens von sauren oder basischen Gruppen in physiologisch verträgliche Salze umwandelt werden (Verfahren c).

Im Verfahren d) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L- Milchsäure sowie (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N-geschützten enantiomeren-reinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze wie Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung von Blutgerinnungsfaktor IXa behandelbar sind. So eignen sich die erfindungsgemäßen Verbindungen als Inhibitoren sowohl für eine prophylaktische als auch für eine therapeutische Anwendung am Menschen. Sie eignen sich sowohl für eine akute Behandlung als auch für eine Langzeittherapie. Die Verbindungen der Formel I können emgesetzt werden in Patienten, die an Störungen des Wohlbefindens oder Krankheiten leiden, die mit Thrombosen, Embolien, Hyperkoagulabilität oder fibrotischen Veränderungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und. Bypass-Operationen. Weiterhin können die Verbindungen der Formel I eingesetzt werden bei allen Eingriffen, die zu einem Kontakt des Blutes mit Fremdoberflächen führen wie bei Dialysepatienten und Patienten mit Verweil-kathetern. Verbindungen der Formel I können auch eingesetzt werden, um die Thrombosegefahr nach chirurgischen Eingriffen wie bei Knie- und Hüftgelenks-operationen zu reduzieren. Verbindungen der Formel I eignen für die Behandlung von Patienten mit disseminierter intravaskulärer Koagulation, Sepsis und anderen intravaskulären Ereignissen, die mit einer Entzündung einhergehen. Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. Verbindungen der Formel I eignen sich zur Verlangsamung oder Verhinderung solcher Prozesse.

Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Eine Beschichtung von Stents mit Verbindungen der Formel I und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines Erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern-, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, Ausb. bedeutet Ausbeute. Verwendete Abkürzungen sind entweder erläutert oder entsprechen den üblichen Konventionen.

Soweit nicht anders angegeben, wurden chromatographische Trennungen an Kieselgel mit Ethylacetat/Heptan-Gemischen als Laufmittel durchgeführt. Präparative Trennungen an Reversed Phase-(RP)-Kieselgel (HPLC) wurden, soweit nicht anders angegeben, unter folgenden Bedingungen durchgeführt: Säule Merck Hibar RT 250-25 LiChrospher 100 RP-18e 5µm, Merck KGaA, Deutschland, Life Science & Analytics, 64293 Darmstadt; mobile Phase A: H₂O + 0,1 % TFA, Phase B: 80% Acetonitril + 0,1 % TFA, Fluss 25 ml/ min., 0 bis 7 min. 100% A , 7 bis 22 min. auf 100% B, 22 bis 30 min. 100% B, 30 bis 33 min. auf 100% A, 33 bis 35 min. 100% A. Das Abdampfen von Lösungsmitteln geschah in der Regel unter vermindertem Druck bei 35 °C bis 45 °C am Rotationsverdampfer. Wenn nicht anders aufgeführt, wurden die LC/MS-Analysen unter folgenden Bedingungen durchgeführt:

| Methode A: | |
|---|---|
| Säule: | YMC J'shere H80 33x2,1 mm; Waters GmbH, Helfmann-Park 10, 65760 Eschborn, Deutschland; Packungsmaterial 4 µm, |
| Lösungsmittel: | ACN+0,05% TFA:H₂O+0.05% TFA (Fluss 1,3 mL/min) |
| Gradient: | 5:95 (0 min) nach 95:5 (2,5 min) nach 95:5 (3,0 min) |
| Ionisierung: | ESI⁺ |
| | |

| Methode B: | |
|---|---|
| Säule: | YMC J'shere H80 33x2,1 mm; Packungsmaterial 4 µm, |
| Lösungsmittel: | ACN+0,05% TFA:H₂O+0,05% TFA (Fluss 1 mL/min) |
| Gradient: | 5:95 (0 min) nach 95:5 (3,4min) nach 95:5 (4,4 min) |
| Ionisierung: | ESI⁺ |
| | |

| Methode D: | |
|---|---|
| Säule: | YMC J'shere ODS H80 20x2,1 mm Packungsmaterial 4 µm, |
| Lösungsmittel: | ACN:H₂O+0,05% TFA (Fluss 1 mL/min) |
| Gradient: | 4:96 (0 min) nach 95:5 (2 min) nach 95:5 (2,4 min) nach 96:4 (2,45 min) |
| Ionisierung: | ESI⁺ |
| | |

| Präparative HPLC wurde mit der folgenden Methode durchgeführt: | |
|---|---|
| | |
| Säule: | Waters Atlantis dC18 OBD 30x100 mm 5µm; Waters GmbH, Helfmann-Park 10, 65760 Eschborn, Deutschland |
| Lösungsmittel: | ACN:H₂O+0,1%TFA (Fluss 60 mL/min) |
| Gradient: | 10 : 90 (0 min) bis 90: 10 (10 min) |
| | |

| Verwendete Abkürzungen: | |
|---|---|
| ACN | Acetonitril |
| Boc | Butoxycarbonyl |
| DCM | Dichlormethan |
| (DHQ)₂PHAL | 1-[(R)-((4S,5R)-5-Ethyl-1-aza-bicyclo[2.2.2]oct-2-yl)-(6-methoxy-chinolin-4-yl)-methoxy]-4-[(R)-((4R,5S)-5-ethyl-1-aza-bicyclo [2.2.2]oct-2-yl)-(6-methoxy-chinolin-4-yl)-methoxy]-phthalazin |
| DIPEA | N,N-Diisopropylethylamin (Hünigs Base) |
| DMF | Dimethylformamid |
| DMSO | Dimethylsulfoxid |
| EDC | N'-(3-Dimethylaminopropyl)-N-ethylcarbodiimid |
| HATU | O-(7-Azabenzotriazol-1-yl)-N,N,N',N'-tetramethyluronium hexafluor-phosphat |
| HOAt | 1-Hydroxy-7-azabenzotriazol |
| K₂[OsO₂(OH)₄] | Kaliumosmat-dihydrat |
| LC/MS | Flüssigkeitschromatographie Massenspektroskopie |
| MeOH | Methanol |
| NMM | N-Methylmorpholin |
| PyBop | 1-Benzotriazolyloxy-tripyrrolidinophosphoniumhexafluor-phosphat |
| PyBrop | Brom-tris-pyrrolidin-phosphonium hexafluorphosphat |
| Rt | Retentionszeit |
| TDBTU | O-(3,4-Dihydro-4-oxo-1,2,3-benzotriazin-3-yl-)N,N,N',N'-tetramethyluronuim-tetrafluoroborat |
| TFA | Trifluoressigsäure |
| TOTU | O-((Ethoxycarbonyl)cyanomethylenemino)-N,N,N',N'-tetramethyluronium tetrafluorborat |
| RT | Raumtemperatur (21 °C bis 24 °C) |

### Beispiel 1

### (2R, 3R)-N-(1-Amino-isochinolin-6-yl)-2,3-dihydroxy-N'-p-tolyl-bernsteinsäureamid; Verbindung mit Trifluoressigsäure

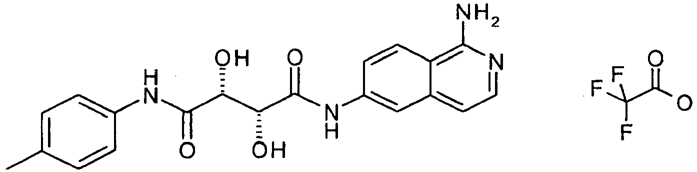

### Verfahrensschritt 1:

### [6-((2R, 3R)-2,3-Dihydroxy-3-p-tolylcarbamoyl-propionylamino)-isochinolin-1-yl]-N-di-carboxyamino tertiär-butylester

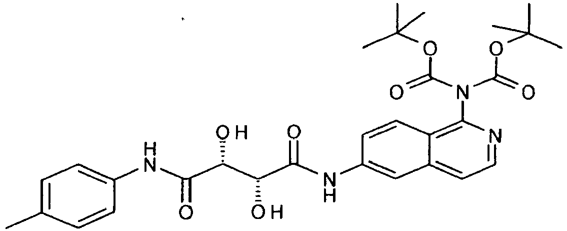

Zu einer Lösung von 26,6 mg (2R,3R)-2,3-dihydroxy-N-p-tolyl-weinsäure (0,111 mmol), 40,0 mg 6-Amino-isochinolin-1-yl)-N- di-carboxyamino tertiär-butylester (0,111 mmol) ((6-Amino-iso-chinolin-1-yl)-N- di-carboxyamino tert-butylester wurde wie in WO2004/072101, Seite 108, beschriebenen Verfahren erhalten) und 15,1 mg HOAt (0,111 mmol) in 1,5 mL DMF wurden 0,037 mL NMM (0,334 mmol) zugefügt und 10 min gerührt. Nach der Zugabe von 52 mg PyBrop (0,111 mmol) wurde das Reaktionsgemisch bei RT für 42 Stunden (h) gerührt. Die Reaktionsmischung wurde filtriert und über eine präparative HPLC gereinigt. Die gereinigten Fraktionen des Produktes wurden lyophylisiert. Es wurden 4 mg eines weißen Feststoffs erhalten.
Ausbeute: 6 % LC/MS (Methode D) (M+H)⁺ 581

### Verfahrensschritt 2:

### (2R, 3R)-N-(1-Amino-isochinolin-6-yl)-2,3-dihydroxy-N'-p-tolyl-bernsteinsäure amid; Verbindung mit Trifluoressigsäure

Zu einer Lösung der Verbindung erhalten aus Verfahrensschritt 1 (4 mg, 6,9 µmol) in 3 mL DCM wurde 1 mL TFA zugefügt und bei RT für 2 h gerührt. Die Lösungsmittel wurden unter verminderten Druck abdestilliert, der Rückstand wurde in MeOH und Wasser gelöst, anschließend wurde über Nacht lyophilisiert und es wurden 4 mg der Titelverbindung als weißer Feststoff erhalten. Reinheit 85 %.
LC/MS (Methode B) 380,15 (Rₜ= 1,05 min, 97%)
¹H NMR (500 MHz, DMSO-d₆) δ (ppm): 1,73 (impurity), 2,26 (s, 3H), 3,01 (impurity), 4,51 (dd, 1 H), 4,57 (dd, 1 H), 6,05 (d, 1 H), 6,22 (d, 1 H), 7,13 (d, 2H), 7,18 (d, 1 H), 7,62 (m, 3H), 8,04 (dd, 1 H), 8,48 (q, 2H), 8,76 (s, 2H), 9,55 (s, 1 H), 10,29 (s, 1H), 12,71 (s, 1 H)

### Beispiel 2:

### (2R, 3R)-N-(2-Amino-3H-benzimidazol-5-yl)-2,3-dihydroxy-N'-p-tolyl-bernsteinsäure-amid; Verbindung mit Trifluoressigsäure

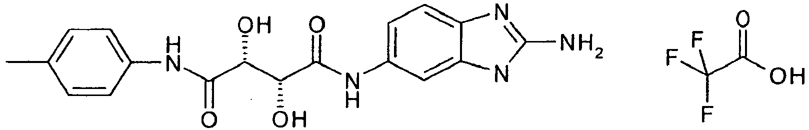

### Verfahrensschritt 1:

### (2R, 3R)-2-Amino-6-(2,3-dihydroxy-3-p-tolylcarbamoyl-propionylamino)-benzimidazole-1- carboxyamino tertiär-butylester

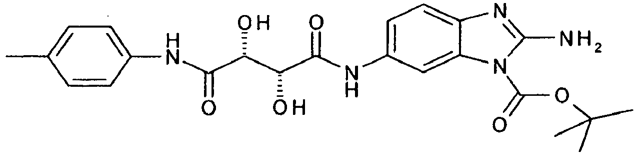

119 mg (2R,3R)-2,3-dihydroxy-N-p-tolyl-weinsäure (0,5 mmol) und 124 mg 2,5-Diamino-benzimidazol-1-carbonsäure-tertiär-butylester (0,5 mmol) (2,5-Diamino-N-boc-benzimidazol-1-carbonsäure-tertiär-butylester wurde gemäß der Internationalen Anmeldung WO2002/042273 hergestellt) wurden in 5 mL von DMF gelöst. 74 mg HOAt (0,55 mmol), 180 mg PyBrop (0,55 mmol) und 193 mg DIPEA (1,5 mmol) wurden in einer Mischung von 1,5 mL DCM und 1,5 mL DMF gelöst und der Reaktionsmischung zugefügt. Nach Rühren über Nacht wurde die Mischung mit Ethylacetat verdünnt. Die organische Phase wurde mit einem wässrigen Natriumhydrogencarbonat Lösung und Kochsalzlösung gewaschen, über Natriumsulfat getrocknet, filtriert und unter verminderten Druck wurden die Lösungsmittel entfernt. Es wurde die Titelverbindung als Öl erhalten.
LC/MS (Methode D) (M+H-tBu)⁺ 414

### Verfahrensschritt 2:

### (2R, 3R)-N-(2-Amino-3H-benzimidazol-5-yl)-2,3-dihydroxy-N'-p-tolyl-bernsteinsäureamid; Verbindung mit Trifluoressigsäure

Der Rückstand aus Verfahrensschritt 1 wurde in einer Mischung aus 2 mL DCM und 2 mL TFA gelöst. Nach Rühren bei RT für 2 h wurden die Lösungsmittel unter verminderten Druck entfernt und mit HPLC gereinigt Es wurden 90 mg (Ausbeute: 37%) der Titelverbindung als weißer Feststoff erhalten.
LC/MS (Methode A) 370,15 (Rₜ= 1,0 min, 100%)
¹H NMR (400 MHz, DMSO-d₆) δ (ppm): 2,23 (s, 3H), 4,47 (t, 2H), 6,00 (dd, 2H), 7,12 (d, 2H), 7,28 (s, 1 H), 7,48 (d, 1 H), 7,62 (d, 2H), 8,03 (s, 1 H), 8,39 (s br, 2H), 9,51 (s, 1 H), 9,78 (s, 1 H)

### Beispiel 3:

### (2R, 3R)-N-(2-Amino-3H-benzimidazol-5-yl)-N'-(4-cyclohexyl-phenyl)-2,3-dihydroxy-bernsteinsäureamid; Verbindung mit Trifluoressigsäure

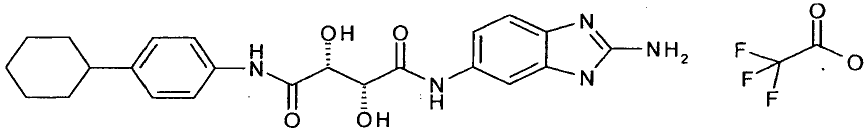

### Verfahrensschritt 1:

### (2R, 3R)-2,3-Diacetoxy-N-(4-cyclohexyl-phenyl)-weinsäure

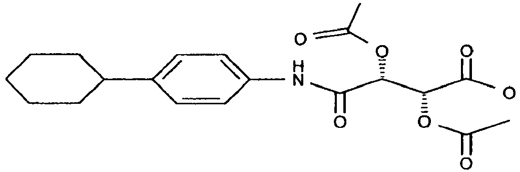

216 mg (+)-Diacetyl-L-weinsäureanhydrid (1 mmol) und 263 mg 4-Cyclohexyl-phenylamin (1,5 mmol) und 200 µL N-Methylmorpholin wurden in 5 mL DMF gelöst und für 3 h bei RT gerührt. Die Mischung wurde mit Ethylacetat verdünnt. Die organische Phase wurde mit 1 N HCl Lösung und Kochsalzlösung gewaschen, über Natriumsulfat getrocknet, filtriert und unter verminderten Druck wurden die Lösungsmittel entfernt. Es wurde 430 mg der Titelverbindung als Öl erhalten (Ausbeute etwa 100 %). LC/MS (Methode D) (M+H)⁺ 392

### Verfahrensschritt 2:

### (2R, 3R)- 2-Amino-6-[2,3-diacetoxy-3-(4-cyclohexyl-phenylcarbamoyl)-propionylamino)-benzimidazol-1-carbonsäure-tertiär-butylester

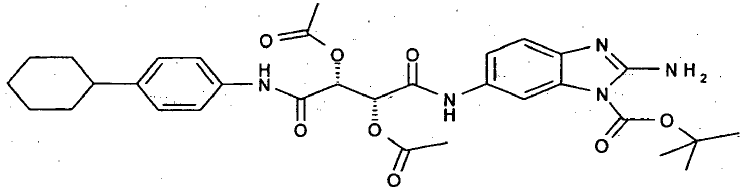

118 mg der Verbindung erhalten aus Verfahrensschritt 1 (0,3 mmol) und 110 µL N-Methylmorpholin wurden in 2 mL DMF bei 0°C gelöst. 115 mg TDBTU (0,33 mmol) wurden zugefügt und es wurde für weitere 30 min gerührt. 82 mg 2,5-Diamino-benzimidazol-1-carbonsäure-tertiär-butylester (0,33 mmol) wurde zugefügt und das Gemisch wurde bei 0°C für 4 h gerührt. Die Mischung wurde mit Ethylacetat verdünnt. Dieorganische Phase wurde mit wässriger Natriumhydrogencarbonat-Lösung und Kochsalzlösung gewaschen, über Natriumsulfat getrocknet, filtriert und unter verminderten Druck wurden die Lösungsmittel entfernt. Es wurde die Titelverbindung als Öl erhalten. LC/MS (Methode D) (M+H)⁺ 622

### Verfahrensschritt 3:

### (2R, 3R)-Essigsäure-2-acetoxy-2-(2-amino-3H-benzimidazol-5-ylcarbamoyl)-1-(4-cyclohexyl- phenylcarbamoyl)-ethylester; Verbindung mit Trifluoressigsäure

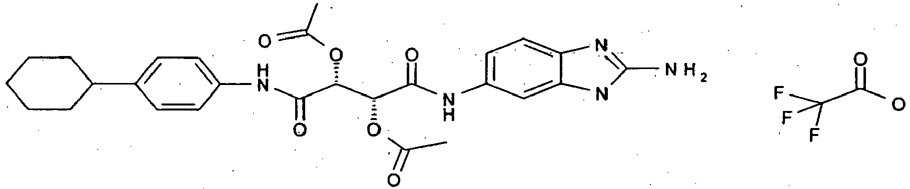

Die Verbindung aus Verfahrensschritt 2 wurde in 3 mL DCM und 1,5 mL TFA gelöst und für 2 h bei RT gerührt. Es wurde die Titelverbindung als Öl erhalten.

### Verfahrensschritt 4:

### (2R, 3R)-N-(2-Amino-3H-benzimidazol-5-yl)-N'-(4-cyclohexyl-phenyl)-2,3-dihydroxy-bernsteinsäureamid; Verbindung mit Trifluoressigsäure

Das Öl aus Verfahrensschritt 3 wurde in 3 mL Methanol gelöst und mit 500 µL 5 N NaOH über Nacht behandelt. Die Lösungsmittel wurden unter verminderten Druck abdestilliert und der Rückstand wurde in 2 mL TFA gelöst und eingedampft. Der Rückstand wurde über eine HPLC gereinigt und es wurde 7 mg der Titelverbindung als weißer Feststoff erhalten. LC/MS (Methode A) 437,2 (Rₜ= 1,39 min, 100%) ¹H NMR (400 MHz, DMSO-d₆) δ (ppm): 1,16-1,42 (m, 5H), 1,68-1,83 (m, 5H), 4,49 (dd, 2H), 5,99 (dd, 2H), 7,17 (d, 2H), 7,25 (d br, 1H), 7,45 (d, 1H), 7,62 (d, 2H), 8,02 (s br, 1 H), 8,36 (s br, 2H), 9,53 (s,1H), 9,78 (s, 1H)

### Beispiel 4:

### (2R,3R)-N-(4-Aminomethyl-phenyl)-2,3-dihydroxy-N'-p-tolyl-bersteinsäureamid; Verbindung mit Trifluoressigsäure

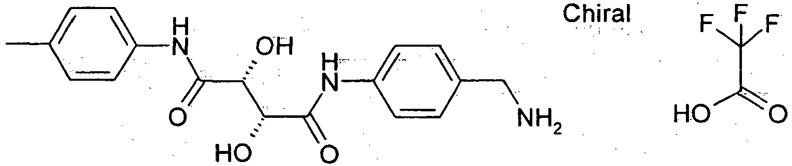

### Verfahrensschritt 1:

### [4-((2R,3R)-2,3-Dihydroxy-3-p-tolyl-carbamoyl-propionylamino)-benzyl]-carbamin säure tert-butyl ester

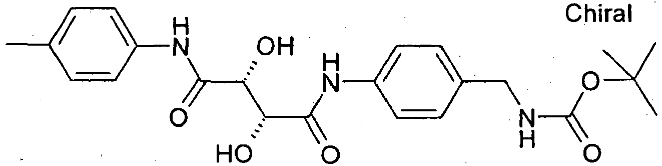

In Analogie zu Beispiel 1, Verfahrensschritt 1 wurden 33 mg (0,15 mmol) (4-Amino-benzyl)-carbaminsäure tert-butyl ester und 36 mg (0,15 mmol) (2R,3R)-2,3-Dihydroxy-N-p-tolyl-bernsteinsäure in 1 ml DCM und 1 ml DMF gelöst und nacheinander mit 78 µl DIPEA (0,45 mmol), 22,5 mg HOAt (0,165 mmol) und 76,9 mg (0,165 mmol) PyBrop versetzt. Ohne weitere Aufarbeitung wurde der erhaltene Ansatz filtriert und mittels HPLC gereinigt. Die produkt-enthaltenden Fraktionen wurden lyophilisiert.

### Verfahrensschritt 2:

### (2R,3R)-N-(4-Aminomethyl-phenyl)-2,3-dihydroxy-N'-p-tolyl-bersteinsäureamid; Verbindung mit Trifluoressigsäure

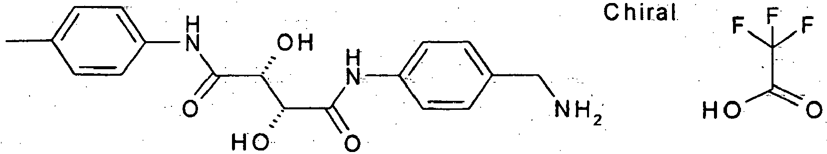

Das Verfahrensprodukt aus Schritt 1 wurde analog zu Beispiel 1; Verfahrensschritt 2, umgesetzt, danach wurde eingeengt, mit Wasser versetzt und lyophilisiert.
Ausbeute: 15,5 mg (23 % über 2 Stufen).
LC/MS (Methode A) M-NH₂= 327,36 (Rₜ=0,88 min, 93%)
¹H NMR (500 MHz, DMSO-d₆) δ (ppm): 2,25 (s, 3H), 3,98 (s br, 2H), 4,49 (m, 2H), 5,99 (d, 1H), 6,09 (d, 1H), 7,12 (d, 2H), 7,39 (d, 2H), 7,62 (d, 2H), 7,79 (d, 2H), 8,12 (s br, 3H), 9,51 (s, 1 H), 9,72 (s, 1H).

### Beispiel 5:

### (2R,3R)-N-(4-Carbamimidoyl-phenyl)-2,3-dihydroxy-N'-p-tolyl-bernsteinsäureamid; Verbindung mit Trifluoressigsäure

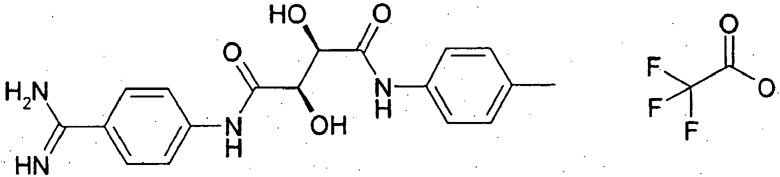

### Verfahrensschritt 1:

### (2R,3R)-2,3-Diacetoxy-N-(4-carbamimidoyl-phenyl)-bersteinsäure; Verbindung mit Trifluoressigsäure

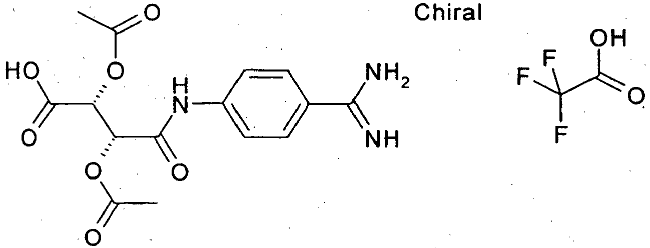

500 mg (2,39 mmol) 4-Amino-benzamidin dihydrochlorid und 519 mg (2,40 mmol) Essigsäure-(3R,4R)-4-acetoxy-2,5-dioxo-tetrahydro-furan-3-yl-ester wurden in 2 ml Pyridin und 2 ml DMF gelöst und mit 25 mg 4-DMAP versetzt. Danach wird für 1 h bei 100 °C erhitzt. Man filtrierte und reinigte mit HPLC. Die produkt-enthaltenden Fraktionen wurden vereinigt und lyophilisiert.
Ausbeute: 130 mg (12%).

### Verfahrensschritt 2:

### (2R,3R)-N-(4-Carbamimidoyl-phenyl)-2,3-dihydroxy-N'-p-tolyl-bernsteinsäureamid; Verbindung mit Trifluoressigsäure

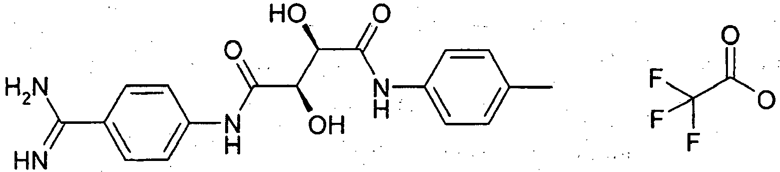

52 mg (0,11 mmol) des in Verfahrensschritt 1 erhaltenen Derivates wurden in 1 ml DMF gelöst und anschließend mit 15 mg (0,13 mmol) Toluidin, 62 mg (0,13 mmol) PyBrop, 18 mg (0,13 mmol) HOAt und 65 µl (0,58 mmol) NMM versetzt. Nach 1 h Rühren bei RT wurden 2 ml MeOH und 90 mg Natriummethylat zugegeben. Nach vollständigem Umsatz wurde filtriert und mittels HPLC gereinigt.
Ausbeute: 19 mg (36 % über 2 Stufen).
LC/MS (Methode A) 357,43 (Rₜ= 0,88 min, 100%)
¹H NMR (500 MHz, DMSO-d₆) δ (ppm): 2,26 (s, 3H), 4,48 (m, 1 H), 4,53 (m, 1 H), 6,01 (m, 1 H), 6,16 (m, 1 H), 7,13 (d, 2H), 7,63 (d, 2H), 7,81 (d, 2H), 8,01 (d, 2H), 8,86 (s br, 2H), 9,20 (s br, 2H), 9,53 (s, 1 H), 10,13 (s, 1 H).

Die Verbindungen in der nachfolgenden Tabelle 1 wurden in analoger Weise wie in den vorstehenden Beispielen hergestellt.

**Tabelle 1:**

| Beispiel Nr. | Struktur | Masse aus LC-MS | Rt aus LC-MS | LC-MS Methode |
|---|---|---|---|---|
| 6 | 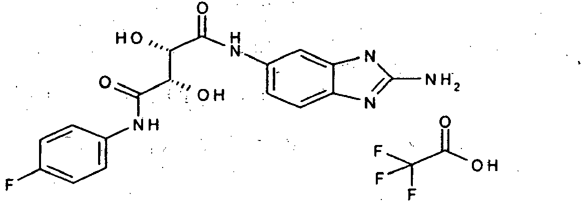 | 374,19 | 0,88 | A |
| 7 | 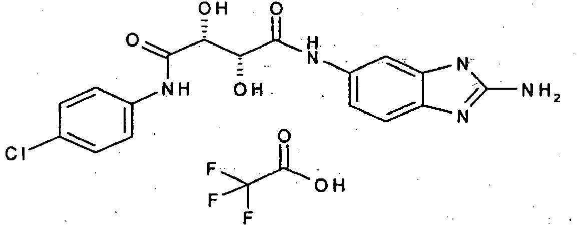 | 390,16 | 1,02 | A |
| 8 | 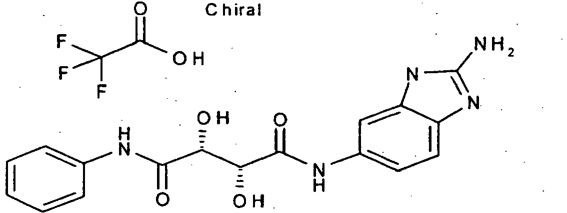 | 356,17 | 0,84 | A |
| 9 | 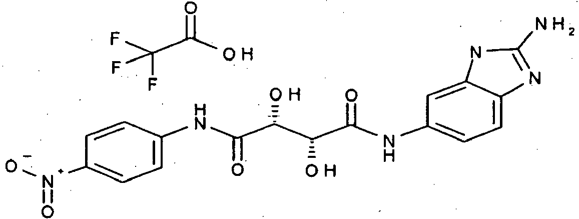 | 401,18 | 0,99 | A |
| 10 | 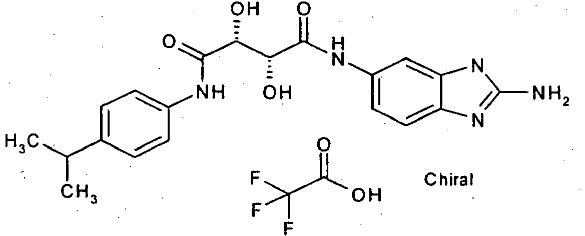 | 398,17 | 1,2 | A |
| 11 | 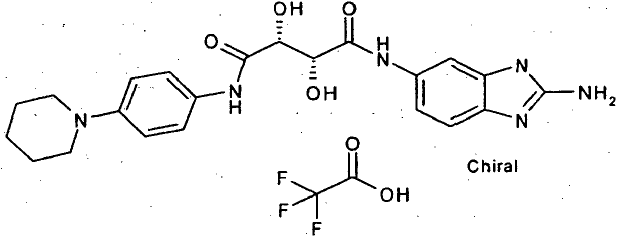 | 439,24 | 0,65 | A |
| 12 | 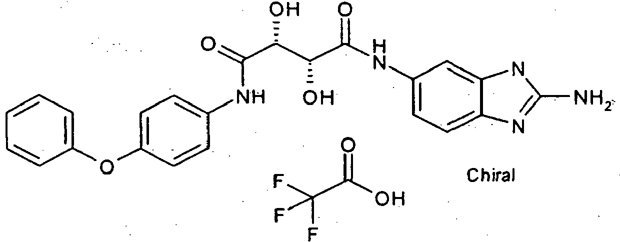 | 448,14 | 1,28 | A |

### Pharmakologische Beispiele

### Faktor IXa Bestimmungsmethode

Die hergestellten Substanzen aus den Beispielen wurden mit dem Substrat PEFA 3107 (Firma Pentapharm/Loxo; über S. Black GmbH, Baumstrasse 41, 47198 Duisburg, Deutschland; Pr. Nr. 095-20) und Faktor IXa (Firma Calbiochem, Merck KGaA vertreibt Calbiochem in Deutschland, Life Science & Analytics, 64293 Darmstadt; Pr. Nr. 233290) auf Inhibition der enzymatischen Aktivität von FIXa geprüft. Dabei wurden zu 2 µl 10 mM Dimethylsulfoxid-Lösung der jeweiligen Testsubstanz, 28 µL Testpuffer (50 mM α,α,α-Tris-(hydroxymethyl)-methylamin (TRIS), 100 mM NaCl, 5 mM CaCl₂, 0,1 % Rinderserumalbumin, pH 7,4) und 10 µL Faktor IXa (277 nM Endkonzentration im Testansatz) gegeben und 15 Minuten bei Raumtemperatur in einer 96 half-well Mikrotiterplatte inkubiert. Die Enzymreaktion wurde durch Zugabe von 10 µL Substrat (1 mM Stocklösung in Wasser) gestartet. Der Zeitverlauf der Reaktion wurde bei 405 nm in einem Mikrotiterplattenreader (SpectraMax plus 384; Firma Molecular Devices) über 15 Minuten verfolgt.

Die IC₅₀ wurde aus den gemittelten Werten (Doppelbestimmung) einer Verdünnungsreihe der Testsubstanz mit Hilfe der Software Grafit 4 (Erithacus Software, UK) berechnet. Die Inhibitions-konstanten (Kᵢ) wurden gemäß der Cheng Prusoff Gleichung Ki = IC₅₀/(1+(S/Km) berechnet, wobei S = Konzentration des Testsubstrats im Test und Kₘ = Michaelis-Menten Konstante bedeutet.
Tabelle 2 zeigt die Ergebnisse.

**Tabelle 2:**

| Verbindung aus Beispiel | Fakto IXa-Enzym-Assay IC₅₀ [mikro M] | Verbindung aus Beispiel | Fakto IXa-Enzym-Assay IC₅₀ [mikro M] |
|---|---|---|---|
| 1 | 0,22 | 12 | 0,37 |
| 5 | 0,17 | | |

## Patentansprüche

1. Verbindung der Formel I 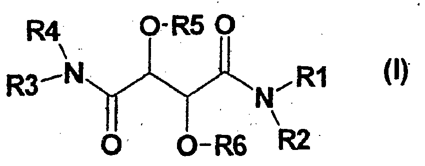 und/oder alle stereoisomeren Formen der Verbindung der Formel I und/oder Gemische dieser Formen in jedem Verhältnis, und/oder ein physiologisch verträgliches Salz der Verbindung der Formel I, wobei
R1 für
1) -(C₆-C₁₄)-Aryl-Z, worin Z eine basische stickstoffhaltige Gruppe bedeutet, wobei die konjugierte Säure dieser Gruppe einen pKa von 5 bis 15 hat und worin Aryl unsubstituiert oder ein-, zwei- oder dreifach durch T substituiert ist,
2) ein vier- bis fünfzehngliedrigen Het-Z, worin Z eine basische stickstoffhaltige Gruppe bedeutet, wobei die konjugierte Säure dieser Gruppe einen pKa von 5 bis 15 hat und worin Het unsubstituiert oder zusätzlich ein-, zwei- oder dreifach durch T substituiert ist, steht,
R2 und R4 gleich sind und jeweils Wasserstoffatom bedeuten,
R3 für
1) -(C₀-C₄)-Alkylen-(C₆-C₁₄)-Aryl, worin Aryl unsubstituiert oder ein-, zwei- oder dreifach durch T substituiert ist,
2) -(C₀-C₄)-Alkylen-(C₆-C₁₄)-Aryl-Q-(C₆-C₁₄)-Aryl, worin die beiden Aryle jeweils unabhängig voneinander unsubstituiert oder ein-, zwei- oder dreifach durch T substituiert sind, oder
3) -(C₀-C₄)-Alkylen-(C₆-C₁₄)-Aryl-Q-Het, worin Aryl und Het jeweils unabhängig voneinander unsubstituiert oder ein-, zwei- oder dreifach durch T substituiert sind, steht,
Q für kovalente Bindung, -(C₁-C₄)-Alkylen, -NH-, -N((C₁-C₄)-Alkyl)-, -O-, -SO₂- oder -S- steht,
T für
1) Halogen,
2) -(C₁-C₆)-Alkyl, worin Alkyl unsubstituiert oder unabhängig voneinander ein, zwei- oder dreifach durch -(C₁-C₃)-Fluoralkyl, -N-C(O)-OH oder -N-C(O)-(C₁-C₄)-Alkyl substituiert sind,
3) -(C₃-C₈)-Cycloalkyl oder
4) -NO₂, steht,
R5 und R6 gleich sind und jeweils für Wasserstoffatom stehen.

2. Verbindung der Formel I gemäß Anspruch 1, wobei
R1 für
1) -(C₆-C₁₄)-Aryl-Z, wobei Aryl ausgewählt ist aus der Gruppe Phenyl und Naphthyl, und worin Aryl unsubstituiert oder ein-, zwei- oder dreifach durch T substituiert ist und Z Amino, Aminomethylen, Amidino, Guanidino, Azetidinyl, Pyrrolidinyl, Piperidinyl, Pyridinyl oder Aminopyridinyl bedeutet, oder
2) ein vier- bis fünfzehngliedrigen Het-Z, wobei Het ausgewählt ist aus der Gruppe Acridinyl, Azepinyl, Azetidinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, beta-Carbolinyl, Chinazolinyl, Chinolinyl, Chinolizinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, Dioxolenyl, 2H, 6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Phenanthridinyl, Phenanthrenyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazinyl, Thiazolyl, Thienyl, Thienoimidazolyl, Thienooxazolyl, Thienopyridinyl; Thienopyrrolyl; Thienothiazolyl, Thienothiophenyl, Thiomorpholinyl, Thiopyranyl, Triazinyl, 1,2,3-Triazolyl, 1,2,4-Triazolyl, 1,2,5-Triazolyl, 1,3,4-Triazolyl oder Xanthenyl und worin Het unsubstituiert oder ein-, zwei- oder dreifach durch T substituiert ist und worin Z wie oben definiert ist, steht,
R2 und R4 gleich sind und jeweils Wasserstoffatom bedeuten,
R3 für
1) -(C₀-C₄)-Alkylen-(C₆-C₁₄)-Aryl, worin Aryl wie oben definiert ist und unsubstituiert oder ein-, zwei- oder dreifach durch T substituiert ist,
2) -(C₀-C₄)-Alkylen-(C₆-C₁₄)-Aryl-Q-(C₆-C₁₄)-Aryl, worin die beiden Aryle jeweils unabhängig voneinander wie oben definiert sind und jeweils unabhängig voneinander unsubstituiert oder ein-, zwei- oder dreifach durch T substituiert sind, oder
3) -(C₀-C₄)-Alkylen-(C₆-C₁₄)-Aryl-Q-Het, worin Aryl und Het wie oben definiert sind und jeweils unabhängig voneinander unsubstituiert oder ein-, zwei- oder dreifach durch T substituiert sind, steht
Q für kovalente Bindung, -(C₁-C₄)-Alkylen, -NH-, -N((C₁-C₄)-Alkyl)- oder -O-steht,
T für
1) Halogen,
2) -(C₁-C₆)-Alkyl, worin Alkyl unsubstituiert oder unabhängig voneinander ein, zwei- oder dreifach durch -(C₁-C₃)-Fluoralkyl, -N-C(O)-OH oder -N-C(O)-(C₁-C₄)-Alkyl substituiert ist,
3) -(C₃-C₆)-Cycloalkyl oder
4) -NO₂, steht,
R5 und R6 gleich sind und jeweils für Wasserstoffatom stehen.

3. Verbindung der Formel I gemäß Anspruch 1, wobei
R2 und R1 für Carbamimidoyl-phenyl, Aminomethylphenyl oder Het-Z steht, wobei Het ausgewählt ist aus der Gruppe Benzimidazolyl und Isochinolinyl, und worin Z für Amino oder Amidino steht, R4 jeweils für Wasserstoffatom stehen,
R3 für
1) Phenyl, worin Phenyl unsubstituiert oder ein- oder zweifach durch T substituiert ist,
2) -Phenyl-Q-Phenyl, worin die beiden Phenylreste jeweils unabhängig voneinander unsubstituiert oder ein- oder zweifach durch T substituiert sind, oder
3) Phenyl-Q-Het-2, worin Het-2 ausgewählt ist aus der Gruppe Chinolinyl, Chinoxalinyl, Furanyl, Indolyl, Isochinolinyl, Isothiazolyl, Isoxazolyl, Oxadiazolyl, Piperidinyl, Pyrazolyl, Pyridazinyl, Pyrimidinyl, Pyridyl, Pyrimidinyl, Pyrrolyl, Thiadiazolyl, Thiazolyl, Thienyl, Thienopyrrol oder Thienothiophenyl, und worin Phenyl und Het-2 jeweils unabhängig voneinander unsubstituiert oder ein- oder zweifach durch T substituiert sind, steht,
Q für kovalente Bindung, -CH₂-, -N(CH₃)- oder -O- steht,
T für
1) F, Cl oder Br,
2) -(C₁-C₄)-Alkyl, worin Alkyl unsubstituiert oder unabhängig voneinander ein- oder zweifach durch -CF₃ oder -N-C(O)-CH₃ substituiert sind, oder
3) -NO₂, steht, und
R5 und R6 jeweils Wasserstoffatom bedeuten.

4. Verbindung der Formel I gemäß einem oder mehreren der Ansprüche 1 bis 3, **dadurch gekennzeichnet, dass** es die Verbindung
(2R, 3R)-N-(1-Amino-isochinolin-6-yl)-2,3-dihydroxy-N'-p-tolyl-bernsteinsäureamid,
(2R, 3R)-N-(2-Amino-3H-benzimidazol-5-yl)-2,3-dihydroxy-N'-p-tolyl-bernsteinsäureamid,
(2R, 3R)-N-(2-Amino-3H-benzimidazol-5-yl)-N'-(4-cyclohexyl-phenyl)-2,3-dihydroxy-bernsteinsäureamid,
(2R,3R)-N-(4-Aminomethyl-phenyl)-2,3-dihydroxy-N'-p-tolyl-bersteinsäure-amid, (2R,3R)-N-(4-Carbamimidoyl-phenyl)-2,3-dihydroxy-N'-p-tolyl-bernsteinsäureamid,
(2R, 3R)-N-(2-Amino-3H-benzimidazol-5-yl)-N'-(4-fluorphenyl)-2,3-dihydroxy-bernsteinsäureamid,
(2R, 3R)-N-(2-Amino-3H-benzimidazol-5-yl)-N'-(4-chlorphenyl)-2,3-dihydroxy-bernsteinsäureamid,
(2R, 3R)-N-(2-Amino-3H-benzimidazol-5-yl)-2,3-dihydroxy-N'-phenyl-bernsteinsäureamid,
(2R, 3R)-N-(2-Amino-3H-benzimidazol-5-yl)-2,3-dihydroxy-N'-(4-nitro-phenyl-bernsteinsäureamid,
(2R,3R)-N-(2-Amino-3H-benzimidazol-5-yl)-2,3-dihydroxy-N'-(4-isopropylphenyl-bernsteinsäureamid,
(2R, 3R)-N-(2-Amino-3H-benzimidazol-5-yl)-2,3-dihydroxy-N'-(4-piperidin-1-yl-phenyl)-bernsteinsäureamid oder
(2R, 3R)-N-(2-Amino-3H-benzimidazol-5-yl)-2,3-dihydroxy-N'-(4-phenoxy-phenyl)-bernsteinsäureamid ist.

5. Verfahren zur Herstellung der Verbindung der Formel I gemäß einem oder mehreren der Ansprüche 1 bis 4, **dadurch gekennzeichnet, dass** man
a) eine Verbindung der Formel II 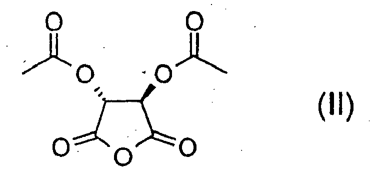 mit einer Verbindung NH(R3)(R4) zu einer Verbindung der Formel III umsetzt, 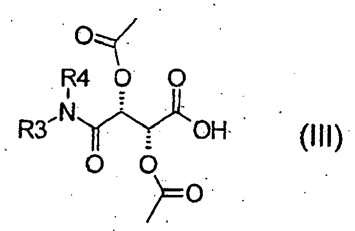 wobei die Reste R3 und R4 wie in Formel I definiert sind und die Verbindung der Formel III mit einer Verbindung NH(R2)(R1)-Boc zu einer Verbindung der Formel VI umsetzt, 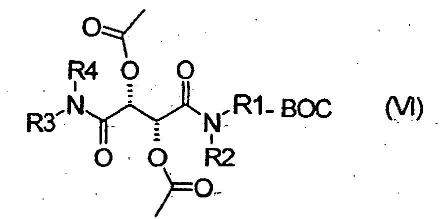 wobei die Reste R1, R2, R3 und R4 wie in Formel I definiert sind und Boc für die Schutzgruppe Butoxycarbonyl steht, und anschließend zu einer Verbindung der Formel I umsetzt, oder
b) eine Verbindung der Formel IV 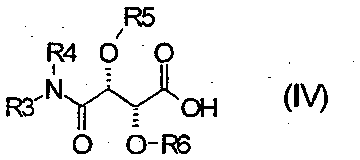 wobei die Reste R3, R4, R5 und R6 wie in Formel I definiert sind, mit einer Verbindung NH(R2)(R1)-Boc zu einer Verbindung der Formel V umsetzt, 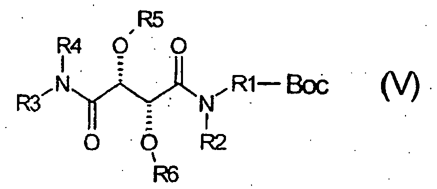 wobei die Reste R1, R2, R3, R4, R5 und R6 wie in Formel I definiert sind und Boc für die Schutzgruppe Butoxycarbonyl steht, und anschließend zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel 1, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

6. Arzneimittel, **gekennzeichnet durch** einen wirksamen Gehalt an mindestens einer Verbindung der Formel I gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

7. Verwendung der Verbindung der Formel I gemäß einem oder mehreren der Ansprüche 1 bis 4 zur Herstellung eines Arzneimittels zur Prophylaxe, Sekundärprävention und Therapie all solcher Erkrankungen, die mit Thrombosen, Embolien, Hyperkoagulabilität oder fibrotischen Veränderungen einhergehen.

8. Verwendung gemäß Anspruch 7, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

## Claims

1. A compound of the formula I 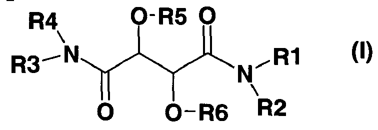 and/or all stereoisomeric forms of the compound of the formula I and/or mixtures of these forms in any ratio, and/or a physiologically tolerable salt of the compound of the formula I, where
R1 is
1) -(C₆-C₁₄)-aryl-Z, in which Z is a basic nitrogen-containing group, where the conjugated acid of this group has a pKa of 5 to 15 and in which aryl is unsubstituted or mono-, di- or trisubstituted by T,
2) a four- to fifteen-membered Het-Z, in which Z is a basic nitrogen-containing group, where the conjugated acid of this group has a pKa of 5 to 15 and in which Het is unsubstituted or additionally mono-, di- or trisubstituted by T,
R2 and R4 are identical and are each a hydrogen atom,
R3 is
1) -(C₀-C₄)-alkylene-(C₆-C₁₄)-aryl, in which aryl is unsubstituted or mono-, di- or trisubstituted by T,
2) -(C₀-C₄)-alkylene-(C₆-C₁₄)-aryl-Q-(C₆-C₁₄)-aryl, in which the two aryls in each case independently of one another are unsubstituted or mono-, di- or trisubstituted by T or,
3) -(C₀-C₄)-alkylene-(C₆-C₁₄)-aryl-Q-Het, in which aryl and Het in each case independently of one another are unsubstituted or mono-, di- or trisubstituted by T,
Q is a covalent bond, -(C₁-C₄)-alkylene, -NH-,-N((C₁-C₄)-alkyl)-, -0-, -SO₂- or -S-,
T is
1) halogen,
2) -(C₁-C₆)-alkyl, in which alkyl are unsubstituted or independently mono-, di- or trisubstituted by -(C₁-C₃)-fluoroalkyl, -N-C(0)-OH or -N-C(O)-(C₁-C₄)-alkyl,
3) -(C₃-C₈)-cycloalkyl, or
4) -NO₂,
R5 and R6 are identical and are in each case a hydrogen atom.

2. A compound of the formula I as claimed in claim 1, where
R1 is
1) -(C₆-C₁₄) -aryl-Z, where aryl is selected from the group consisting of phenyl and naphthyl, and in which aryl is unsubstituted or mono-, di- or trisubstituted by T and Z is amino, aminomethylene, amidino, guanidino, azetidinyl, pyrrolidinyl, piperidinyl, pyridinyl or aminopyridinyl, or
2) a four- to fifteen-membered Het-Z, where Het is selected from the group consisting of acridinyl, azepinyl, azetidinyl, benzimidazolinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, carbazolyl, 4aH-carbazolyl, carbolinyl, beta-carbolinyl, quinazolinyl, quinolinyl, quinolizinyl, 4H-quinolizinyl, quinoxalinyl, quinuclidinyl, chromanyl, chromenyl, cinnolinyl, deca-hydroquinolinyl, dibenzofuranyl, dibenzothiophenyl, dihydrofuran[2,3-b]-tetrahydrofuranyl, dihydrofuranyl, dioxolyl, dioxanyl, dioxolenyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isoquinolinyl, isochromanyl, isoindazolyl, isoindolinyl, isoindolyl, isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxothiolanyl, phenanthridinyl, phenanthrenyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridothiophenyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazinyl, thiazolyl, thienyl, thienoimidazolyl, thienooxazolyl, thienopyridinyl, thienopyrrolyl, thienothiazolyl, thienothiophenyl, thiomorpholinyl, thiopyranyl, triazinyl, 1,2,3-triazolyl, 1,2,4-triazolyl, 1,2,5-triazolyl, 1,3,4-triazolyl or xanthenyl and in which Het is unsubstituted or mono-, di- or trisubstituted by T and in which Z is as defined above,
R2 and R4 are identical and are each a hydrogen atom,
R3 is
1) -(C₀-C₄)-alkylene-(C₆-C₁₄)-aryl, in which aryl is as defined above and is unsubstituted or mono-, di- or trisubstituted by T,
2) -(C₀-C₄)-alkylene-(C₆-C₁₄)-aryl-Q-(C₆-C₁₄)-aryl, in which the two aryls in each case independently of one another are as defined above and in each case independently of one another are unsubstituted or mono-, di- or trisubstituted by T, or
3) -(C₀-C₄)-alkylene-(C₆-C₁₄)-aryl-Q-Het, in which aryl and Het are as defined above and in each case independently of one another are unsubstituted or mono-, di- or trisubstituted by T,
Q is a covalent bond, -(C₁-C₄)-alkylene, -NH-,-N((C₁-C₄)-alkyl)- or -O-,
T is
1) halogen,
2) -(C₁-C₆)-alkyl, in which alkyl is unsubstituted or independently mono-, di- or trisubstituted by -(C₁-C₃)-fluoroalkyl, -N-C(O)-OH or -N-C(O)-(C₁-C₄)-alkyl,
3) -(C₃-C₆)-cycloalkyl, or
4) -NO₂,
R5 and R6 are identical and are each a hydrogen atom.

3. A compound of the formula I as claimed in claim 1, where
R1 is carbamimidoylphenyl, aminomethylphenyl or Het-Z, where Het is selected from the group consisting of benzimidazolyl and isoquinolinyl, and in which Z is amino or amidino;
R2 and R4 are in each case a hydrogen atom,
R3 is
1) phenyl, in which phenyl is unsubstituted or mono- or disubstituted by T,
2) -phenyl-Q-phenyl, in which the two phenyl radicals in each case independently of one another are unsubstituted or mono- or disubstituted by T or
3) phenyl-Q-Het-2, in which Het-2 is selected from the group consisting of quinolinyl, quinoxalinyl, furanyl, indolyl, isoquinolinyl, isothiazolyl, isoxazolyl, oxadiazolyl, piperidinyl, pyrazolyl, pyridazinyl, pyridyl, pyrimidinyl, pyrrolyl, thiadiazolyl, thiazolyl, thienyl, thienopyrrolyl or thienothiophenyl, and in which phenyl and Het-2 in each case independently of one another are unsubstituted or mono- or disubstituted by T,
Q is a covalent bond, -CH₂-, -N(CH₃)- or -O-,
T is
1) F, Cl or Br,
2) -(C₁-C₄)-alkyl, in which alkyl are unsubstituted or independently mono- or disubstituted by -CF₃ or -N-C(O)-CH₃, or
3) -NO₂, and
R5 and R6 are in each case a hydrogen atom.

4. A compound of the formula I as claimed in one or more of claims 1 to 3, which is the compound
(2R, 3R)-N-(1-aminoisoquinolin-6-yl)-2,3-dihydroxy-N'-p-tolylsuccinamide,
(2R, 3R)-N-(2-amino-3H-benzimidazol-5-yl)-2,3-dihydroxy-N'-p-tolyl-succinamide,
(2R, 3R)-N-(2-amino-3H-benzimidazol-5-yl)-N'-(4-cyclohexylphenyl)-2,3-dihydroxysuccinamide,
(2R,3R)-N-(4-aminomethylphenyl)-2,3-dihydroxy-N'-p-tolylsuccinamide,
(2R,3R)-N-(4-carbamimidoylphenyl)-2,3-dihydroxy-N'-p-tolylsuccinamide,
(2R, 3R)-N-(2-amino-3H-benzimidazol-5-yl)-N'-(4-fluorophenyl)-2,3-dihydroxy-succinamide,
(2R, 3R)-N-(2-amino-3H-benzimidazol-5-yl)-N'-(4-chlorophenyl)-2,3-dihydroxysuccinamide,
(2R, 3R)-N-(2-amino-3H-benzimidazol-5-yl)-2,3-dihydroxy-N'-phenyl-succinamide,
(2R, 3R)-N-(2-amino-3H-benzimidazol-5-yl)-2,3-dihydroxy-N'-(4-nitrophenyl)-succinamide,
(2R, 3R)-N-(2-amino-3H-benzimidazol-5-yl)-2,3-dihydroxy-N'-(4-isopropyl-phenyl)succinamide,
(2R, 3R)-N-(2-amino-3H-benzimidazol-5-yl)-2,3-dihydroxy-N'-(4-piperidin-1-yl-phenyl)succinamide or
(2R, 3R)-N-(2-amino-3H-benzimidazol-5-yl)-2,3-dihydroxy-N'-(4-phenoxy-phenyl)succinamide.

5. A process for the preparation of the compound of the formula I as claimed in one or more of claims 1 to 4, which comprises
a) reacting a compound of the formula II 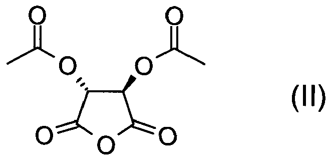 with a compound NH(R3)(R4) to give a compound of the formula III, 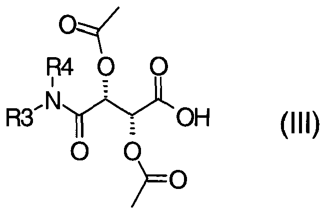 where the radicals R3 and R4 are as defined in formula I, and reacting the compound of the formula III with a compound NH(R2)(R1)-Boc to give a compound of the formula VI, 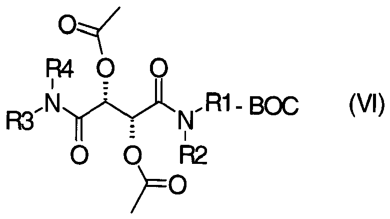 where the radicals R1, R2, R3 and R4 are as defined in formula I and Boc is the protective group butoxycarbonyl, and subsequently reacting to give a compound of the formula I, or
b) reacting a compound of the formula IV 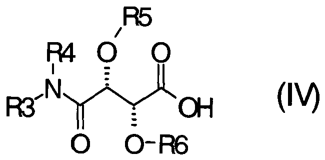 where the radicals R3, R4, R5 and R6 are as defined in formula I, with a compound NH(R2)(R1)-Boc to give a compound of the formula V, 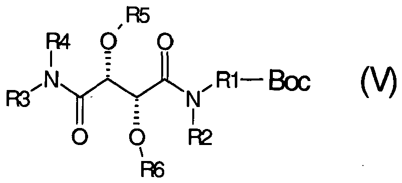 where the radicals R1, R2, R3, R4, R5 and R6 are as defined in formula I and Boc is the protective group butoxycarbonyl, and subsequently reacting to give a compound of the formula I, or
c) either isolating the compound of the formula I prepared according to process a) or b) in free form or releasing it from physiologically intolerable salts or, in the case of the presence of acidic or basic groups, converting it into physiologically tolerable salts, or
d) separating a compound of the formula I prepared according to process a) or b), or a suitable precursor of the formula I which on account of its chemical structure occurs in enantiomeric or diastereomeric forms, into the pure enantiomers or diastereomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary groups.

6. A medicament which comprises an efficacious amount of at least one compound of the formula I as claimed in one or more of claims 1 to 4 together with a pharmaceutically suitable and physiologically tolerable vehicle, additive and/or other active substances and auxiliaries.

7. The use of the compound of the formula I as claimed in one or more of claims 1 to 4 for the production of a medicament for the prophylaxis, secondary prevention and therapy of all those diseases which accompany thromboses, embolisms, hypercoagulability or fibrotic changes.

8. The use as claimed in claim 7, wherein the disease is a myocardial infarct, angina pectoris and other forms of acute coronary syndrome, stroke, peripheral vascular diseases, deep vein thrombosis, pulmonary embolism, embolic or thrombotic events caused by cardiac arrhythmias, cardiovascular events such as restenosis after revascularization and angioplasty and similar interventions such as stent implantations and bypass operations, or the reduction of the risk of thrombosis after surgical interventions such as in knee and hip joint operations, or disseminated intravascular coagulation, sepsis and other intravascular events which accompany inflammation, atherosclerosis, diabetes and the metabolic syndrome and their sequelae, tumor growth and tumor metastasis, inflammatory and degenerative joint diseases such as rheumatoid arthritis and arthrosis, disorders of the hemostatic system such as fibrin deposits, fibrotic changes of the lungs such as chronic obstructive pulmonary disease, adult respiratory distress syndrome or fibrin deposits in the eye after eye operations or prevention and/or treatment of scar formation.

## Revendications

1. Composé de formule I 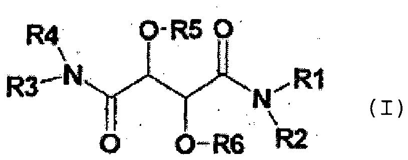 et/ou toutes formes stéréoisomères du composé de formule I et/ou tous mélanges de ces formes en tout rapport, et/ou sel physiologiquement acceptable du composé de formule I, dans laquelle
R1 représente
1) un groupe -aryl(C₆-C₁₄)-Z, dans lequel Z représente un groupe azoté basique, l'acide conjugué de ce groupe ayant un pKa de 5 à 15, et dans lequel le fragment aryle est non substitué ou une, deux ou trois fois substitué par T ;
2) un groupe Het-Z à quatre à quinze chaînons, dans lequel Z représente un groupe azoté basique, l'acide conjugué de ce groupe ayant un pKa de 5 à 15, et dans lequel Het est non substitué ou additionnellement une, deux ou trois fois substitué par T,
R2 et R4 sont identiques et représentent chacun un atome d'hydrogène,
R3 représente
1) un groupe -alkylène(C₀-C₄)-aryle(C₆-C₁₄), dans lequel le fragment aryle est non substitué ou une, deux ou trois fois substitué par T,
2) un groupe -alkylène (C₀-C₄) -aryl (C₆-C₁₄) -Q-aryle(C₆-C₁₄), dans lequel les deux fragments aryle sont chacun, indépendamment l'un de l'autre, non substitués ou une, deux ou trois fois substitués par T, ou
3) un groupe -alkylène (C₀-C₄)-aryl (C₆-C₁₄) -Q-Het, dans lequel les fragments aryle et Het sont chacun, indépendamment l'un de l'autre, non substitués ou une, deux ou trois fois substitués par T,
Q représente une liaison covalente, un groupe -alkylène(C₁-C₄), -NH-, -N(alkyle(C₁-C₄))-, -O-, -SO₂- ou -S-,
T représente
1) des atomes d'halogène,
2) des groupes -alkyle(C₁-C₆), les groupes alkyle étant non substitués ou une, deux ou trois fois substitués, indépendamment les uns des autres, par -fluoroalkyle(C₁-C₃), -N-C(O)-OH ou -N-C(O)-alkyle (C₁-C₄) ,
3) des groupes -cycloalkyle(C₃-C₈) ou
4) -NO₂ ;
R5 et R6 sont identiques et représentent chacun un atome d'hydrogène.

2. Composé de formule I selon la revendication 1, dans lequel
R1 représente
1) un groupe -aryl (C₆-C₁₄) -Z, le fragment aryle étant choisi dans le groupe constitué par phényle et naphtyle, et dans lequel le fragment aryle est non substitué ou une, deux ou trois fois substitué par T et Z représente le groupe amino, aminométhylène, amidino, guanidino, azétidinyle, pyrrolidinyle, pipéridinyle, pyridinyle ou aminopyridinyle,
2) un groupe Het-Z à quatre à quinze chaînons, Het étant choisi dans le groupe constitué par acridinyle, azépinyle, azétidinyle, benzimidazolinyle, benzimidazolyle, benzofuranyle, benzothiofuranyle, benzothiophényle, benzoxazolyle, benzothiazolyle, benzotriazolyle, benzotétrazolyle, benzisoxazolyle, benzisothiazolyle, carbazolyle, 4aH-carbazolyle, carbolinyle, bêta-carbolinyle, quinazolinyle, quinolinyle, quinolizinyle, 4H-quinolizinyle, quinoxalinyle, quinuclidinyle, chromanyle, chroményle, cinnolinyle, décahydroquinolinyle, dibenzofuranyle, dibenzothiophényle, dihydrofurane[2,3-b]-tétrahydrofuranyle, dihydro-furanyle, dioxolyle, dioxanyle, dioxolényle, 2H,6H-1,5,2-dithiazinyle, furanyle, furazanyle, imidazolidinyle, imidazolinyle, imidazolyle, 1H-indazolyle, indolinyle, indolizinyle, indolyle, 3H-indolyle, isobenzofuranyle, isoquinolinyle, isochromanyle, iso-indazolyle, iso-indolinyle, iso-indolyle, isothiazolidinyle, 2-iso-thiazolinyle, isothiazolyle, isoxazolyle, isoxazolidinyle, 2-isoxazolinyle, morpholinyle, naphtyridinyle, octahydro-isoquinolinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, oxothiolanyle, phénanthridinyle, phénanthrényle, phénanthrolinyle, phénazinyle, phénothiazinyle, phénoxathiinyle, phénoxazinyle, phtalazinyle, pipérazinyle, pipéridinyle, ptéridinyle, purinyle, pyranyle, pyrazinyle, pyrazolidinyle, pyrazolinyle, pyrazolyle, pyridazinyle, pyrido-oxazolyle, pyrido-imidazolyle, pyridothiazolyle, pyridothiophényle, pyridyle, pyrimidinyle, pyrrolidinyle, pyrrolinyle, 2H-pyrrolyle, pyrrolyle, tétrahydrofuranyle, tétrahydro-isoquinolinyle, tétrahydroquinolinyle, tétrahydro-pyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazinyle, thiazolyle, thiényle, thiéno-imidazolyle, thiéno-oxazolyle, thiénopyridinyle, thiénopyrrolyle, thiénothiazolyle, thiénothiophényle, thio-morpholinyle, thiopyranyle, triazinyle, 1,2,3-triazolyle, 1,2,4-triazolyle, 1,2,5-triazolyle, 1,3,4-triazolyle ou xanthényle et dans lequel Het est non substitué ou une, deux ou trois fois substitué par T et dans lequel Z est tel que défini plus haut,
R2 et R4 sont identiques et représentent chacun un atome d'hydrogène,
R3 représente
1) un groupe -alkylène (C₀-C₄) -aryle (C₆-C₁₄), dans lequel le fragment aryle est tel que défini plus haut et est non substitué ou une, deux ou trois fois substitué par T,
2) un groupe -alkylène(C₀-C₄)-aryl(C₆-C₁₄)-Q-aryle(C₆-C₁₄), dans lequel les deux fragments aryle sont chacun, indépendamment l'un de l'autre, tels que définis plus haut et sont chacun, indépendamment l'un de l'autre, non substitués ou une, deux ou trois fois substitués par T, ou
3) un groupe -alkylène (C₀-C₄)-aryl (C₆-C₁₄)-Q-Het, dans lequel les fragments aryle et Het sont tels que définis plus haut et sont chacun, indépendamment l'un de l'autre, non substitués ou une, deux ou trois fois substitués par T,
Q représente une liaison covalente, un groupe -alkylène(C₁-C₄), -NH-, -N(alkyle(C₁-C₄))- ou -O-,
T représente
1) un atome d'halogène,
2) un groupe -alkyle(C₁-C₆), le groupe alkyle étant non substitué ou, chaque fois indépendamment, une, deux ou trois fois substitué par -fluoroalkyle(C₁-C₃), -N-C(O)-OH ou -N-C(O)-alkyle (C₁-C₄),
3) un groupe -cycloalkyle(C₃-C₆) ou
4) -NO₂;
R5 et R6 sont identiques et représentent chacun un atome d'hydrogène.

3. Composé de formule I selon la revendication 1, dans lequel
R1 représente un groupe carbamimidoyl-phényle, aminométhylphényle ou Het-Z, Het étant choisi dans le groupe constitué par benzimidazoyle et isoquinolinyle, et Z représentant un groupe amino ou amidino,
R2 et R4 représentent chacun un atome d'hydrogène,
R3 représente
1) un groupe phényle, le groupe phényle étant non substitué ou une ou deux fois substitué par T,
2) un groupe -phényl-Q-phényle, dans lequel les deux radicaux phényle sont chacun, indépendamment l'un de l'autre, non substitués ou une ou deux fois substitués par T, ou
3) un groupe phényl-Q-Het-2 dans lequel Het-2 est choisi dans le groupe constitué par quinolinyle, quinoxalinyle, furanyle, indolyle, isoquinolinyle, isothiazolyle, isoxazolyle, oxadiazolyle, pipéridinyle, pyrazolyle, pyridazinyle, pyridyle, pyrimidinyle, pyrrolyle, thiadiazolyle, thiazolyle, thiényle, thiénopyrrole ou thiénothiophényle, et dans lequel les groupes phényle et Het-2 sont chacun, indépendamment l'un de l'autre, non substitués ou une ou deux fois substitués par T,
Q représente une liaison covalente, un groupe -CH₂-, -N(CH₃)- ou -O-,
T représente
1) F, Cl ou Br,
2) des groupes -alkyle(C₁-C₄), les groupes alkyle étant non substitués ou, indépendamment les uns des autres, une ou deux fois substitués par -CF₃ ou -N-C(O)-CH₃, ou
3) -NO₂, et
R5 et R6 sont identiques et représentent chacun un atome d'hydrogène.

4. Composé de formule I selon une ou plusieurs des revendications 1 à 3, **caractérisé en ce que** le composé est
le (2R,3R)-N-(1-amino-isoquinolin-6-yl)-2,3-dihydroxy-N'-p-tolyl-succinamide,
le (2R,3R)-N-(2-amino-3H-benzimidazol-5-yl)-2,3-dihydroxy-N'-p-tolyl-succinamide,
le (2R,3R)-N-(2-amino-3H-benzimidazol-5-yl)-N'-(4-cyclohexyl-phényl)-2,3-dihydroxy-succinamide,
le (2R,3R)-N-(4-aminométhyl-phényl)-2,3-dihydroxy-N'-p-tolyl-succinamide,
le (2R,3R)-N-(4-carbamimidoyl-phényl)-2,3-dihydroxy-N'-p-tolyl-succinamide,
le (2R,3R)-N-(2-amino-3H-benzimidazol-5-yl)-N'-(4-fluorophényl)-2,3-dihydroxy-succinamide,
le (2R,3R)-N-(2-amino-3H-benzimidazol-5-yl)-N'-(4-chlorophényl)-2,3-dihydroxy-succinamide,
le (2R,3R)-N-(2-amino-3H-benzimidazol-5-yl)-2,3-dihydroxy-N'-phényl-succinamide,
le (2R,3R)-N-(2-amino-3H-benzimidazol-5-yl)-2,3-dihydroxy-N'-(4-nitro-phényl)-succinamide,
le (2R,3R)-N-(2-amino-3H-benzimidazol-5-yl)-2,3-dihydroxy-N'-(4-isopropyl-phényl)-succinamide,
le (2R,3R)-N-(2-amino-3H-benzimidazol-5-yl)-2,3-dihydroxy-N'-(4-pipéridin-1-yl-phényl)-succinamide ou
le (2R,3R)-N-(2-amino-3H-benzimidazol-5-yl)-2,3-dihydroxy-N'-(4-phénoxy-phényl)-succinamide.

5. Procédé pour la préparation du composé de formule I selon une ou plusieurs des revendications 1 à 4, **caractérisé en ce que**
a) on fait réagir un composé de formule II 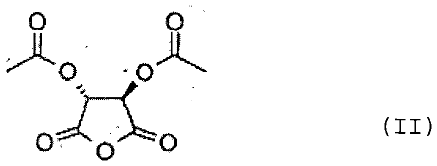 avec un composé NH(R3) (R4) pour aboutir à un composé de formule III, 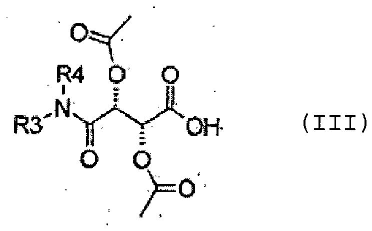 dans laquelle les radicaux R3 et R4 sont tels que définis dans la formule I, et on fait réagir le composé de formule III avec un composé NH(R2)(R1)-Boc pour aboutir à un composé de formule VI, 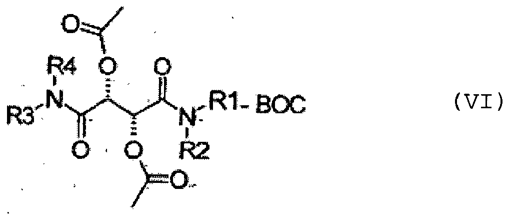 dans laquelle R1, R2, R3 et R4 sont tels que définis dans la formule I et Boc représente le groupe protecteur butoxycarbonyle, et ensuite on le convertit en un composé de formule I, ou
b) on fait réagir un composé de formule IV 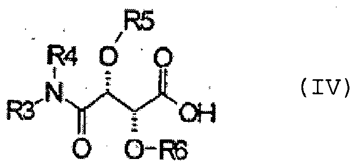 dans laquelle les radicaux R3, R4, R5 et R6 sont tels que définis dans la formule I, avec un composé NH(R2)(R1)-Boc pour aboutir à un composé de formule V, 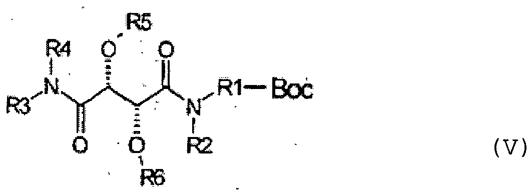 dans laquelle les radicaux R1, R2, R3, R4, R5 et R6 sont tels que définis dans la formule I et Boc représente le groupe protecteur butoxycarbonyle, et ensuite on le convertit en un composé de formule I, ou
c) soit on isole sous forme libre le composé de formule I préparé selon le procédé a) ou b), soit on le libère à partir de sels physiologiquement acceptables ou, dans le cas de la présence de groupes acides ou basiques on le convertit en sels physiologiquement acceptables, ou
d) on résout en les énantiomères ou diastéréoisomères purs un composé de formule I préparé selon le procédé a) ou b), ou un précurseur approprié de formule I, qui en raison de sa structure chimique apparaît sous des formes énantiomères ou diastéréoisomères, par salification avec des acides ou des bases sous forme d'énantiomères purs, chromatographie sur des phases stationnaires chirales ou transformation en dérivé au moyen de composés chiraux sous forme d'énantiomères purs, tels que des acides aminés, séparation des diastéréoisomères ainsi obtenus, et élimination des groupes auxiliaires chiraux.

6. Médicament, **caractérisé par** une teneur efficace en au moins un composé de formule I selon une ou plusieurs des revendications 1 à 4, conjointement avec un véhicule, additif et/ou d'autres principes actifs et adjuvants, pharmaceutiquement appropriés et physiologiquement acceptables.

7. Utilisation du composé de formule I selon une ou plusieurs des revendications 1 à 4, pour la fabrication d'un médicament destiné à la prophylaxie, la prévention secondaire et la thérapie de toutes les maladies qui s'accompagnent de thromboses, d'embolies, d'hypercoagulabilité ou d'altérations fibreuses.

8. Utilisation selon la revendication 7, **caractérisée en ce qu'**il s'agit d'infarctus du myocarde, d'angine de poitrine et d'autres formes du syndrome coronarien aigu, de l'accident vasculaire cérébral, des maladies vasculaires périphériques, de la thrombose des veines profondes, de l'embolie pulmonaire, d'incidents emboliques ou thrombotiques dus à des arythmies cardiales, d'incidents cardiovasculaires tels que la resténose après revascularisation et angioplastie et interventions similaires telles qu'implantation de stents et opérations de pontage, ou pour la réduction du risque de thrombose après des interventions chirurgicales comme dans des opérations des articulations du genou et de la hanche, ou **en ce qu'**il s'agit de la coagulation intravasculaire disséminée, du sepsis, et d'autres incidents intravasculaires qui s'accompagnent d'une inflammation, de l'athérosclérose, du diabète et du syndrome métabolique et de leurs suites, de la croissance tumorales et de la dissémination de métastases tumorale, d'arthropathies inflammatoires et dégénératives telles que la polyarthrite rhumatoïde et l'arthrose, de troubles du système hémostatique tels que des dépôts de fibrine, d'altérations fibreuses du poumon telles que la pneumopathie obstructive chronique, le syndrome de détresse respiratoire de l'adulte ou de dépôts de fibrine dans l'oeil après des opérations des yeux ou pour éviter et/ou traiter la formation de cicatrices.
